# EUROPEAN PATENT APPLICATION

(11) **EP 3 903 690 A1**
(43) Date of publication of application: **03.11.2021**
(21) Application number: 19902007.4
(22) Date of filing: 26.12.2019
(51) Int. Cl.: A61B 17/02, A61B 17/88

(54) **SPREADER**

(30) Priority: 27.12.2018 JP 2018244498; 28.08.2019 JP 2019156090
(71) Applicant: Kuraray Co., Ltd., Kurashiki-shi, Okayama 710-0801 (JP)
(72) Inventor: KUWAYAMA Tomoya, Tokyo 100-8115 (JP)
(74) Representative: Müller-Boré & Partner Patentanwälte PartG mbB
(86) International application number: PCT/JP2019/051056
(87) International publication number: WO 2020/138255

(57) **Abstract**

To provide a spreader that can easily and smoothly open and close a plurality of blades collectively or individually and that has a simple configuration with excellent operability and durability. The spreader includes a spreader body (30) including a first blade (10) and a second blade (20), and a drive member (50). Each of the first and second blades (10, 20) includes a pair of arms (11a, 11b, 21a, 21b) and an angle adjuster (12, 22). An opening and closing mechanism (40) includes a pair of shafts (41a, 41b). Each of the shafts (41a, 41b) includes a handle (46a, 46b) and an operation portion (47a, 47b). Each of the operation portions (47a, 47b) includes an engagement protrusion (48a, 48b) that engages with an engagement recess (134a, 134b, 234a, 234b) of a base end portion (14a, 24a) in the first blade (10) and the second blade (20).

## Description

The present disclosure relates to a spreader.

### BACKGROUND ART

As a treatment for knee osteoarthritis, high tibial osteotomy (High Tibial Osteotomy: HTO) for correction of genu varum (i.e., bow legs), distal femoral osteotomy (Distal Femoral Osteotomy: DFO) for correction of genu valgum (i.e., knock-knee), or the like is performed.

For such osteotomies, various technologies have been developed to form a space to which an implant such as an artificial bone is inserted. The space is formed by widening or enlarging a cut section (osteotomy section) formed in deformed femur or tibia (see Patent Literatures I to VI and Non-Patent Literature I, for example).

Patent Literature I discloses a spreader including a blade consisting of a pair of swinging members (arms) that are rotatably connected at tips thereof, and a pair of opening and closing mechanisms that open and close the blade.

Patent Literature II discloses a spreader including two blades that are detachably connected, and two opening and closing mechanisms that open and close the blades. Each of the blades includes a pair of elongate swinging members that are rotatably connected at tips thereof. The swinging members of one of the blades include protrusions (engaging portions) that engage with recesses in the swinging members of the other blade when the blades are connected.

Patent Literatures III to VI disclose a spreader including two blades and an opening and closing mechanism that collectively opens and closes the two blades. Each of the two blades includes a pair of elongate swinging members that are rotatably connected at tips thereof.

In Patent Literature I, an artificial bone is inserted into the cut section, which has been enlarged by the swinging members, with the swinging members inserted thereinto. Accordingly, the swinging members may interfere with the insertion of the artificial bone.

On the other hand, in the invention disclosed in Patent Literature II, after enlarging the cut section by the two blades inserted thereto, the protrusion and the recess are disengaged and the second blade is removed from the cut section to leave the space. Then, the artificial bone is inserted into the space. Thereby, the artificial bone is easily inserted into the cut section.

However, the invention disclosed in Patent Literature II, the second blade is removed only after closing the second blade, separating the first and second blades from each other in a direction intersecting the insert direction to the cut section, and disengaging the protrusion and the recess. Accordingly, the artificial bone may not be set quickly, and as a result, the time required to implant the artificial bone may be relatively long. In addition, when the correction angle (opening angle) between the blades is relatively small, the protrusion and the recess may not be completely disengaged when the second blade is closed and accordingly the protrusion and the recess may interfere with each other, which makes it difficult to separate the blades.

Also, in the spreader disclosed in Patent Literature II, when the rotation angles of the blades are adjusted, the rotation angle of the first blade is adjusted by applying an operation force thereto, which also adjusts the rotation angle of the second blade engaged with the first blade. However, the blades are easily disengaged since the blades are engaged with each other only by the protrusions and the recesses. Accordingly, when the blades are disengaged unexpectedly, the operation force from the first blade is not sufficiently transmitted to the second blade, which makes it difficult to stably adjust the rotation angles of the blades to the same angle. Moreover, a relatively large load is applied to the engaging parts between the protrusions and the recesses, which may affect the durability of the spreader.

On the other hand, in the inventions disclosed in Patent Literatures III to VI, two blades are linked by a connection member and opened and closed collectively by one opening and closing mechanism. Accordingly, it is possible to suppress the load from being concentrated on specific parts of the blades.

However, in the inventions disclosed in Patent Literatures III to VI, the opening and closing mechanism is operated by rotating an elongate screwdriver or a hexagon wrench at a location far away from the blades. Accordingly, the force may not properly be transmitted to the opening and closing mechanism. Further, an excessive load may be applied to a connection portion between the screwdriver or the hexagon wrench and the opening and closing mechanism, which may affect the durability thereof. Also, the connecting member that connects the swinging members is inserted into the cut section by being struck or driven with a plastic hammer or the like, which makes it difficult to apply the striking force in a well-balanced manner. Also, in the inventions disclosed in Patent Literatures III, V, and VI, the cut section is enlarged by linking the two blades, and accordingly, it is not suitable for a case where the opening angles of the front and rear parts of the osteotomy section are adjusted separately as in Non-Patent Literature I. In addition, the invention disclosed in Patent Literature IV requires a complicated operating mechanism with gears for opening and closing the spreader and a driver, which increases the number of the components.

### CITATION LIST

### Patent Literature

Patent Literature I: JP 4468635B
Patent Literature II: JP 4736091B
Patent Literature III: JP 2016-209435A
Patent Literature IV: JP 2017-46783A
Patent Literature V: JP 2018-175828A
Patent Literature VI: JP 2018-192066A

### Non-Patent Literature

Non Patent Literature I: Japan Knee Osteotomy Forum ed., May 25, 2018, "Zero Kara Hajimeru! Knee Osteotomy Update", ZEN•NIHONBYOIN•SHUPPANKAI, 191-196

### SUMMARY

### Technical Problem

The present disclosure has been made considering the above issues, and an object of the present disclosure is to provide a spreader that can easily and smoothly open and close a plurality of blades collectively or individually and that has a simple configuration with excellent operability and durability.

### Solution to Problem

To achieve the above object, a spreader of the present disclosure includes a spreader body that comprises a first blade and a second blade that is arranged in parallel with the first blade, and an opening and closing mechanism that is configured to open and close the first blade and the second blade. Each of the first blade and the second blade includes a first arm and a second arm that are connected at tips to rotate in a direction away from each other and a direction close to each other, and an angle adjuster that is configured to adjust an angle between the first arm and the second arm. The opening and closing mechanism includes a plurality of shafts that are rotatably connected by a connection shaft. Each of the plurality of shafts includes a handle at a first end and an operation portion at a second end. The operation portion is configured to be operated in conjunction with the handle. The connection shaft is located between the first end and the second end. The operation portion of each of the plurality of shafts includes an engagement portion that is configured to engage with a base end portion of the first arm or the second arm of the first blade and/or a base end portion of the first arm or the second arm of the second blade.

### Advantageous Effects

The present disclosure can provide the spreader that collectively or separately opens and closes a plurality of blades easily and smoothly with a simple structure and provides excellent operability and durability.

### BRIEF DESCRIPTION OF DRAWINGS

[FIG. 1] FIG. 1 is a plan view illustrating components of a spreader of a first embodiment.
[FIG. 2A] FIG. 2A is a side view illustrating first and second blades of FIG. 1 arranged in parallel adjacent to each other (set screw not shown).
[FIG. 2B] FIG. 2B is a cross-sectional view illustrating the first blade shown in FIG. 1.
[FIG. 3A] FIG. 3A is a plan view illustrating a spreader body and a drive member attached thereto.
[FIG. 3B] FIG. 3B is a side view illustrating the spreader body and the drive member attached thereto (set screw not shown).
[FIG. 4A] FIG. 4A is a plan view illustrating the spreader body and an opening and closing mechanism attached thereto.
[FIG. 4B] FIG. 4B is a side view illustrating the spreader body and the opening and closing mechanism attached thereto (set screw not shown).
[FIG. 5] FIG. 5 is a plan view illustrating the first and second blades of the spreader body which are opened by the opening and closing mechanism.
[FIG. 6A] FIG. 6A is a plan view illustrating arms of the first blade which are held in the open state by an angle adjuster.
[FIG. 6B] FIG. 6B is a plan view illustrating the arms of the first and second blades which are held in the open state by the angle adjuster.
[FIG. 7] FIG. 7. is a view illustrating the second blade removed from a cut section.
[FIG. 8A] FIG. 8A is a side view illustrating first and second blades of a spreader of a second embodiment which are arranged in parallel adjacent to each other (set screw not shown).
[FIG. 8B] FIG. 8B is a plan view illustrating the first blade of the spreader of the second embodiment.
[FIG. 9] FIG. 9 is a plan view illustrating a spreader body of the spreader of the second embodiment which is in the open state with the opening and closing mechanism attached to the spreader body.
[FIG. 10A] FIG. 10A is a plan view illustrating first and second opening and closing mechanisms of a spreader of a third embodiment which are arranged in parallel adjacent to each other.
[FIG. 10B] FIG. 10B is a side view illustrating the first and second opening and closing mechanisms of the spreader of the third embodiment which are separated from each other.
[FIG. 11A] FIG. 11A is a plan view illustrating the spreader body of the spreader of the third embodiment to which the first and second opening and closing mechanisms are attached.
[FIG. 11B] FIG. 11B is a side view illustrating the spreader body of the spreader of the third embodiment to which the first and second opening and closing mechanisms are attached (set screw not shown).
[FIG. 12] FIG. 12 is a plan view illustrating the first blade of the spreader body of the third embodiment which is opened by the first opening and closing mechanism.
[FIG. 13A] FIG. 13A is a plan view illustrating first and second opening and closing mechanisms of a spreader of a fourth embodiment which are arranged in parallel adjacent to each other and connected by an engagement mechanism.
[FIG. 13B] FIG. 13B is a side view illustrating the first and second opening and closing mechanisms of the spreader of the fourth embodiment which are separated from each other.
[FIG. 14A] FIG. 14A is a view illustrating a first operation portion which is opened by operating a first handle of an opening and closing mechanism of a spreader of a fifth embodiment.
[FIG. 14B] FIG. 14B is an A-arrow view of FIG. 14A illustrating the opening and closing mechanism of the spreader of the fifth embodiment in the closed state.
[FIG. 14C] FIG. 14C is a B-arrow view of FIG. 14A illustrating the opening and closing mechanism of the spreader of the fifth embodiment in the closed state.
[FIG. 15A] FIG. 15A is a plan view illustrating first and second opening and closing mechanisms of a spreader of a sixth embodiment which are arranged in parallel adjacent to each other.
[FIG. 15B] FIG. 15B is a side view illustrating the first and second opening and closing mechanisms of the spreader of the sixth embodiment which are separated from each other.
[FIG. 16A] FIG. 16A is a plan view illustrating first and second opening and closing mechanisms of a spreader of a seventh embodiment which are arranged in parallel and connected by an engagement mechanism.
[FIG. 16B] FIG. 16B is a side view illustrating the first and second opening and closing mechanisms of the spreader of the seventh embodiment which are separated from each other.
[FIG. 17A] FIG. 17A is a plan view illustrating first and second opening and closing mechanisms of a spreader of an eighth embodiment which are arranged in parallel adjacent to each other.
[FIG. 17B] FIG. 17B is a side view illustrating the first and second opening and closing mechanisms of the spreader of the eighth embodiment which are separated from each other.
[FIG. 18A] FIG. 18A is a plan view illustrating first and second opening and closing mechanisms of a spreader of a ninth embodiment which are arranged in parallel and connected by an engagement mechanism.
[FIG. 18B] FIG. 18B is a side view illustrating the first and second opening and closing mechanisms of the spreader of the ninth embodiment which are separated from each other.
[FIG. 19A] FIG. 19A is a plan view illustrating first and second opening and closing mechanisms of a spreader of a tenth embodiment which are arranged in parallel and positioned by a positioning portion.
[FIG. 19B] FIG. 19B is a side view illustrating the first and second opening and closing mechanisms of the spreader of the tenth embodiment which are separated from each other.

### DESCRIPTION OF EMBODIMENTS

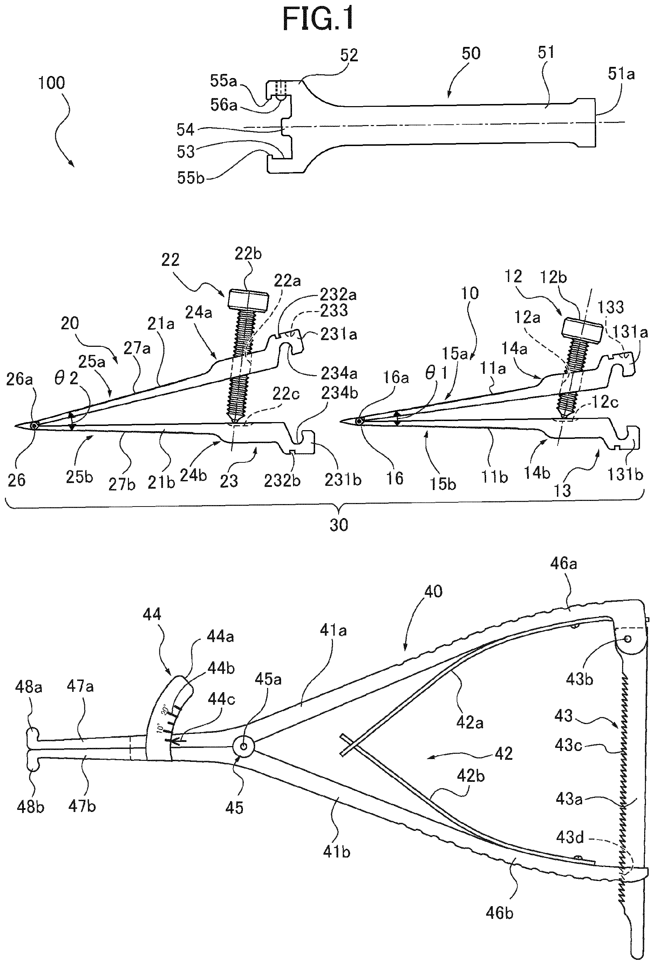

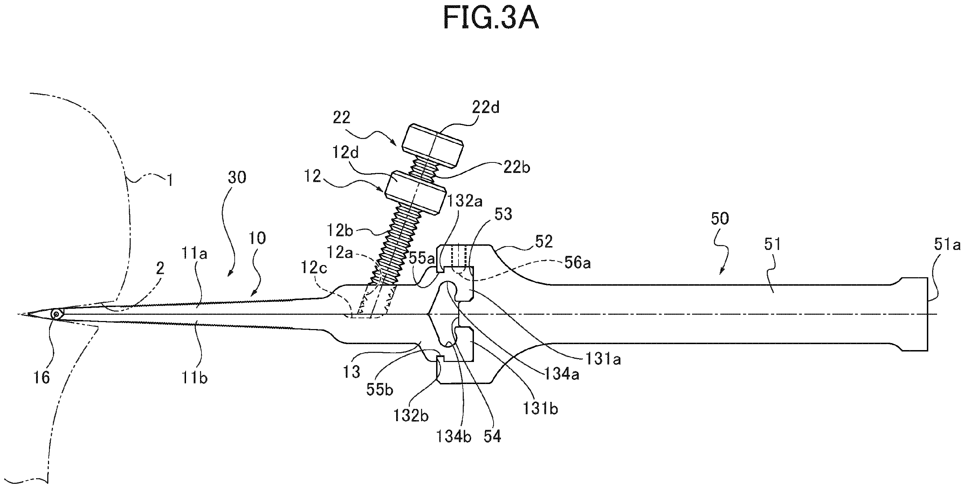

(First Embodiment) Hereinafter, a spreader according to a first embodiment of the present disclosure will be described with reference to the drawings. FIG. 1 is a plan view illustrating components of the spreader 100 of the first embodiment. As shown in FIG. 1, the spreader 100 of the first embodiment includes a spreader body 30 including a first blade 10 and a second blade 20, and an opening and closing mechanism (widening handle) 40 that opens and closes the first and second blades 10, 20. In addition, the spreader 100 of the first embodiment includes a strike or drive member (drive handle) 50 for striking or driving the first and second blades 10, 20 into a cut section 2 formed in a bone (see FIG. 3A, etc.)

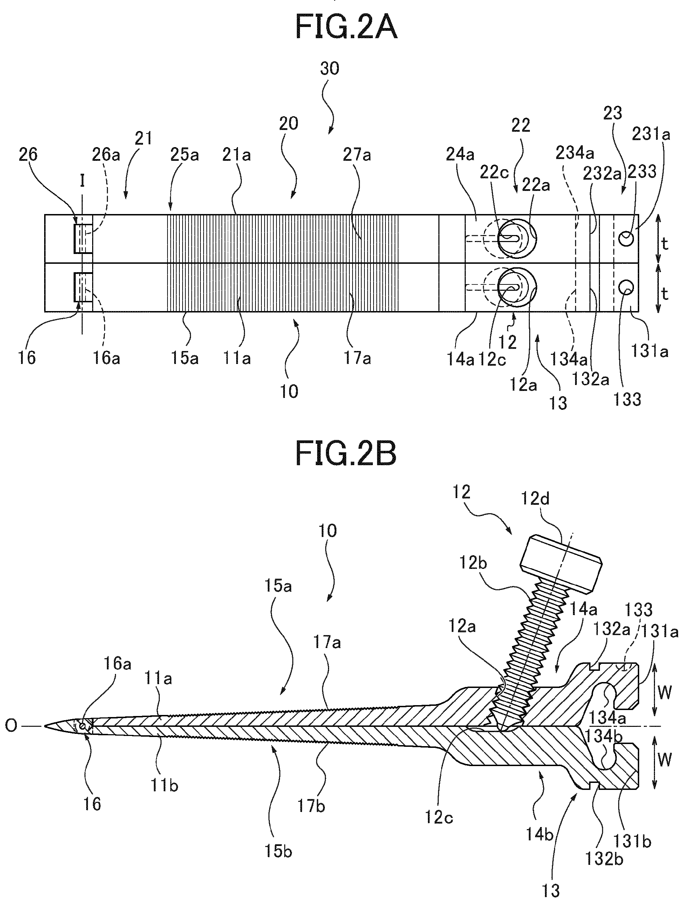

The spreader 100 of this embodiment is used for the surgery of a bone 1 such as the high tibial osteotomy (HTO) or the like, for example. The spreader 100 is configured to be inserted into the cut section 2 with the first and second blades 10, 20 of the spreader body 30 arranged in parallel to each other (see FIG. 2A) and enlarge or spread the cut section 2 to form a space in which an implant such as the artificial bone is inserted.

It should be noted that the "spreader" herein is defined as one that includes the spreader body 30 including the first and second blades, the opening and closing mechanism 40, and optionally the drive member 50 as described above. However, the "spreader" of the present disclosure is not limited to the above spreader. For example, the spreader body 30 may be defined as the "spreader". Also, a configuration or system including the "spreader", the opening and closing mechanism 40, and optionally the drive member 50 may be defined as a "spreader set".

For ease of explanation in the specification, a side closer to the cut section 2 is referred to as a "tip or tip side" while a hand side of a practitioner such as a doctor, a surgeon, or the like who operates the spreader 100 opposite to the tip is referred to as "base or base end". Also, an extending direction of the spreader body 30 (direction parallel to center line O shown in FIG. 2B) is referred to as a "longitudinal direction", and an opening direction of the spreader body 30 is referred to as a "width direction". A direction perpendicular to "the longitudinal direction" and "the width direction" (direction of axis I of connection shafts 16a, 26a shown in FIG. 2A) is referred to as a "height direction" or an "arrangement direction". Among a pair of surfaces of each member, a surface facing outward is referred to as an "outer surface" while a surface facing inward is referred to as an "inner surface". Also, in the plan view of FIG. 1, a front side of the drawing is referred to as a "front side" and a back or rear side of the drawing is referred to as a "back or rear side".

As shown in the side views of FIGS. 1 and 2A as well as the cross-sectional view of FIG. 2B, the blade 10 includes a pair of (first and second) arms 11a, 11b. Each of the arms 11a, 11b includes a base end portion 14a, 14b to which the opening and closing mechanism 40 and the drive member 50 are attached, and an insert portion 15a, 15b which is inserted into the cut section 2 of the bone 1. Each of the base end portions 14a, 14b includes an angle adjuster 12 and an engaged portion 13.

Tips of the arms 11a, 11b are rotatably connected by a connection portion 16. In this embodiment, the connection portion 16 includes a pin-shaped connection shaft 16a and a bearing that rotatably supports the connection shaft 16a, and has a so-called pivot structure. According to this structure, the arms 11a, 11b are rotatable in a direction away from and a direction close to each other with the connection shaft 16a as the axis of rotation. It should be noted that the connection portion 16 is not limited to the one having the pivot structure and may have a known structure.

Each of the arms 11a, 11b consists of an elongate metal member. The thickness "t" of the arm in the height direction shown in FIG. 2A is constant from the tip to the base end. According to this structure, the arms 11a, 11b have excellent strength against the driving or striking and opening operations. The first and second blades 10, 20 are arranged or stacked in parallel in the direction of axis I (height direction).

On the other hand, each of the arms 11a, 11b has the width "w" shown in FIG. 2B where the base end portion 14a, 14b is wider than the insert portions 15a, 15b. According to this structure, the base end portions 14a, 14b have excellent strength against the drive or striking and opening operations. The width of the insert portion 15a, 15b gradually decreases from the side facing the base end portion 14a, 14b to the tip to have a tapered shape (wedge shape). According to the tapered shape, the insert portions 15a, 15b can be easily inserted into the cut section 2.

An angle between the outer surfaces of the arms 11a, 11b in the closed state (also simply referred to as "angle between the arms 11a, 11b", etc. hereinafter) is five degrees. That is, the initial value (minimum value) of an angle θ1 between the arms 11a, 11b is five degrees. The same applied to an angle θ2 between the arms 21a, 21b of the second blade 20. It should be noted that the minimum values of the angles θ1, θ2 are not limited to five degrees. The minimum values of the angles θ1, θ2 may be set as desired by appropriately changing the inclined angles of the arms 11a, 11b, 21a, 21b depending on the type of surgery, the site of surgery, the angle of the correction, the size of the implant or the like.

The angle adjuster 12 is configured to adjust the angle θ1 between the arms 11a, 11b, which open and close via the connection portion 16, and maintain the adjusted angle θ1. The angle adjuster 12 includes a screw hole 12a formed in the base end portion 14a of the arm 11a and a set screw 12b that is inserted into the screw hole 12a. The screw hole 12a extends through the base end portion 14a from the outer surface to the inner surface thereof.

As shown in FIG. 2B, the arms 11a, 11b can be closed when the set screw 12b is removed from the screw hole 12a or the set screw 12b is set so that the tip does not extend from the inner surface. On the other hand, the arms 11a, 11b can be maintained in the open state, and the unexpected closing movement of the arms 11a, 11b can be prevented by rotating the set screw 12b in an engaging direction (direction screw hole 12a moves forward) such that the tip of the set screw 12b extends from the screw hole 12a to abut the inner surface of the second arm 11b when the arms 11a, 11b are opened. Also, the angle θ1 between the arms 11a, 11b can be adjusted by adjusting the amount of insertion of the set screw 12b into the screw hole 12a.

The tip of the set screw 12b has a hemispherical shape. The inner surface of the second arm 11b which the tip of the set screw 12b contacts is provided with a recessed groove 12c. The recessed groove 12c extends in the longitudinal direction of the second arm 11b to receive the tip of the set screw 12b. According to this structure, the tip of the set screw 12b can reliably contact the inner surface of the second arm 11b and the support for the second arm 11b by the set screw 12b can be more stable even when the inclination angles of the arms 11a, 11b change relatively.

The outer surfaces of the arms 11a, 11b are provided with slip stoppers 17a, 17b, respectively. Each of the slip stoppers 17a, 17b includes a plurality of grooves that extends in a direction interesting the insert direction to the cut section 2. The slip stoppers 17a, 17b bite into the internal tissue or the like of the bone 1, which prevents the first blade 10 inserted into the cut section 2 from unexpectedly falling or being removed.

As shown in FIGS. 2A and 2B, the engaged portion 13 includes a pair of engagement protrusions 131a, 131b, a pair of engagement grooves 132a, 132b, a ball receiver 133, and a pair of engagement recesses 134a, 134b.

The engagement protrusions 131a, 131b are provided at the base end side of the base end portions 14a, 14b to extend in the width direction. The engagement grooves 132a, 132b are provided parallel to the axis I in the outer surfaces of the engagement protrusions 131a, 131b, respectively. Each of the engagement grooves 132a, 132b is rectangular in plan view. The ball receiver 133 is formed by recessing the outer surface of the engagement protrusion 131a in a hemispherical shape.

As shown in FIG. 2B, the engagement recesses 134a, 134b are provided in the inner surfaces of the base end portion 14a, 14b, respectively. The engagement recesses 134a, 134b have a substantially T-shape (or substantially arrow shape) in a plan view when the arms 11a, 11b are closed. This shape can properly prevent below-described engagement protrusions 48a, 48b of the opening and closing mechanism 40 engaged with the engagement recesses 134a, 134b from being unexpectedly removed.

The engagement protrusions 131a, 131b are received by a receiving recess 53 of the drive member 50, which is described below. The engagement grooves 132a, 132b engage with engagement ridges 55a, 55b of the drive member 50. The ball receiver 133 engages with a ball plunger 56A of the drive member 50. The engagement recesses 134a, 134b mainly engage with the engagement protrusions 48a, 48b of the opening and closing mechanism 40, which is described below. Also, the engagement recesses 134a, 134b engage with an engagement protrusion 54 of the drive member 50.

The second blade 20 has a basic configuration similar to the first blade 10 except that an elongate set screw 22b which is longer than the set screw 12b of the first blade 10 is provided for the second blade 20. The shapes and dimensions of the elements of the second blade 20 are the same as those of the first blade 10 except the set screw 12b. However, the set screw 22b may be the same as the set screw 12b, which can simplify the manufacturing process without distinguishing the first and second blades 10, 20.

In this way, having the same configuration, the first blade 10 and the second blade 20 can be manufactured in the same process using the same mold or die and the like, which improves productivity thereof. Hereinafter, the elements of the second blade 20 will be briefly described and detailed explanations are omitted.

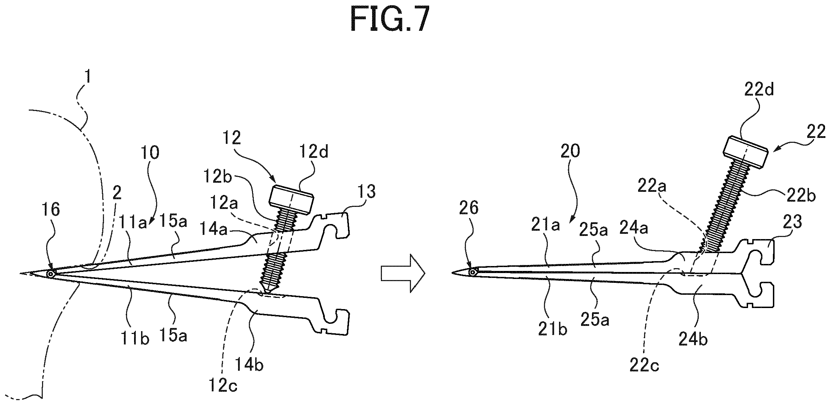

As shown in FIGS. 1 and 7, the second blade 20 includes arms 21a, 21b. Each of the arms 21a, 21b includes a base end portion 24a, 24b and an insert portion 25a, 25b, respectively. The base end portions 24a, 24b include an angle adjuster 22 and an engaged part 23.

The tips of the arms 21a, 21b are rotatably connected by a connection portion 26. The connection portion 26 includes a connection shaft 26a and a bearing and has a pivot structure. The thickness of each of the arms 21a, 21b in the height direction is constant from the tip to the base end. The thickness of each of the base end portions 24a, 24b in the width direction is larger than that of each of the insert portions 25a, 25b. Each of the insert portions 25a, 25b becomes gradually thinner from the base end portion 24b side to the tip to have a tapered shape (wedge shape). The outer surfaces of the arms 21a, 21b are respectively provided with slip stoppers 27a, 27b which are grooves.

The angle adjuster 22 is configured to adjust the angle θ2 between the arms 21a, 21b. The angle adjuster 22 includes a screw hole 22a provided in the base end portion 24a of the first arm 21a, the set screw 22b, and a recessed groove 22c. As described above, the set screw 22b is longer than the set screw 12b. Thereby, head portions 12d, 22d of the set screws 12b, 22B are offset and do not interfere with each other when the first and second blades 10, 20 are arranged in parallel adjacent to each other as shown in FIG. 3A. Accordingly, the set screws 12b, 22b can be easily operated.

It should be noted that the first and second blades 10, 20 may be arranged such that the set screws 12b, 22b extend in different directions. In this case, the surgeon can access the set screws 12b, 22b from two directions to rotate them. The arms 11a, 11b, 21a, 21b of the first and second blades 10, 20 are formed line-symmetrically with a center line O therebetween. Therefore, the angle adjusters 12, 22 may be arranged in the same orientation or in different orientations depending on a part to be operated and the ease of operation for the surgeon.

The engaged part 23 includes a pair of engagement protrusions 231a, 231b, a pair of engagement grooves 232a, 232b, a ball receiver 233, and a pair of engagement recesses 234a, 234b.

The opening and closing mechanism 40 is configured to open and close the first and second blades 10, 20 of the spreader body 30 at one time. The opening and closing mechanism 40 includes a pair of shafts 41a, 41b, a biasing member 42, a ratchet mechanism 43, and an angle scale member (first angle scale member) 44. The shafts 41a, 41b are rotatably connected by a connection portion 45. For example, the connection portion 45 has a pivot structure that includes a connection shaft 45a and a bearing. The shafts 41a, 41b respectively include handles 46a, 46b at the base end side, and operation portions 47a, 47b at the tip side with the connection shaft 45a located therebetween. The operation portions 47a, 47b are opened and closed in conjunction with the opening and closing movements of the handles 46a, 46b.

Each of the operation portions 47a, 47b has an L-shape in a plan view. Each of the operation portions 47a, 47b respectively includes the engagement protrusion 48a, 48b, at the tip thereof, that extends in the width direction perpendicular to the longitudinal direction. The first engagement protrusion 48a engages with the first engagement recesses 134a, 234a of the first and second blades 10, 20 while the second engagement recess 48b engages with the second engagement recesses 134b, 234b of the first and second blades 10, 20.

The biasing member 42 is configured to bias the handles 46a, 46b in directions away from each other. For example, the biasing member 42 consists of a pair of leaf springs 42a, 42b. In the state that the handles 46a, 46b are biased by the biasing member 42 in directions away from each other, the operation portions 47a, 47b are closed as shown in FIG. 1.

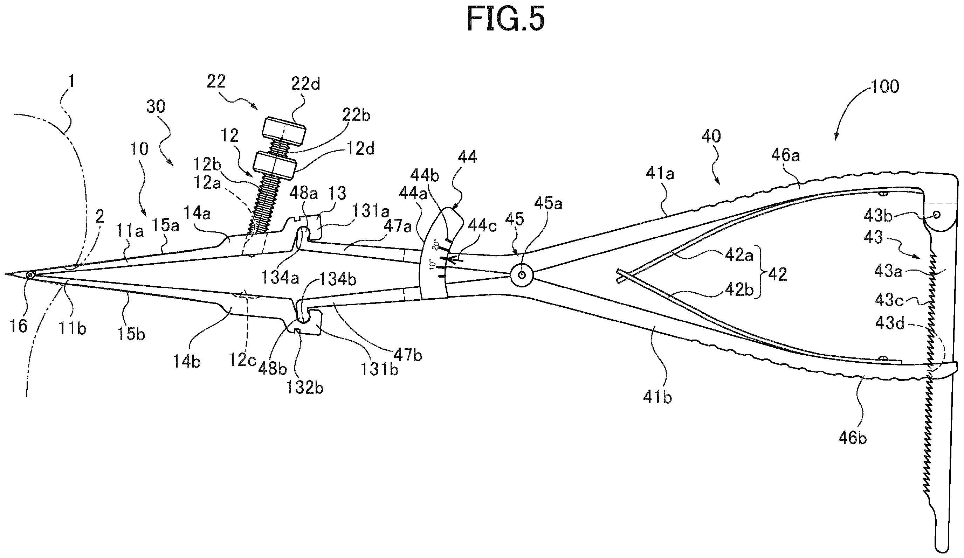

On the other hand, when the handles 46a, 46b are moved in the directions close to each other against the biasing force of the biasing member 42, the operation portions 47a, 47b rotate about the connection shaft 45a in directions away from each other so that the operation portions 47a, 47b are opened (see FIG. 5).

The ratchet mechanism 43 is provided at the base end of the handles 46a, 46b and configured to control the opening and closing movements of the handles 46a, 46b (i.e., opening and closing movements of operation portions 47a, 47b). The ratchet mechanism 43 includes a lever 43a, a connection shaft 43b, and a teeth stopper 43d. The lever 43a includes a plurality of ratchet teeth 43c. The connection shaft 43b supports one end of the lever 43a at an end of the first handle 46a. The teeth stopper 43d is disposed at an end of the second handle 46b to engage with the ratchet teeth 43c.

With the ratchet mechanism 43 configured as above, the handles 46a, 46b are allowed to move in the directions close to each other but not allowed to move in the directions away from each other since the ratchet teeth 43c engage with the teeth stopper 43d. When the lever 43a is rotated about the connection shaft 43b in a disengaging direction from the teeth stopper 43d to disengage the teeth stopper 43d from the ratchet teeth 43c, the biasing force of the biasing member 42 moves the handles 46a, 46b in the directions away from each other so that the handles 46a, 46b return to the original positions.

The angle scale member 44 is configured to indicate the angle between the operation portions 47a, 47b (opening angle) and indirectly indicate the angles between the arms 11a, 11b, 21a, 21b of the spreader body to the surgeon or practitioner. As shown in FIG. 1, the angle scale member 44 includes a plate 44a, angle scale marks 44b and an arrow-shaped needle 44c. The plate 44a is provided on the front surface of the operation portion 47b of the second shaft 41b. The angle scale marks 44b are provided on the front surface of the plate 44a. The arrow-shaped needle 44c is provided on the front surface of the operation portion 47b of the first shaft 41a to point to one of the angle scale marks 44b.

One end of the plate 44a is fixed to the second shaft 41b. The plate 44a has an arc shape with the connection shaft 45a as a center and extends toward the first shaft 41a. With this configuration, the angle between the operation portions 47a, 47b, which rotate about the connection shaft 45a, can be measured accurately.

In this embodiment, as mentioned above, each of the angles θ1, θ2 between the arms 11a, 11b, 21a, 21b is five degrees when the arms are closed. Accordingly, the needle 44c points to a five-degree mark of the angle scale marks 44b when the shafts 41a, 41b are closed (see FIG. 1, etc.).

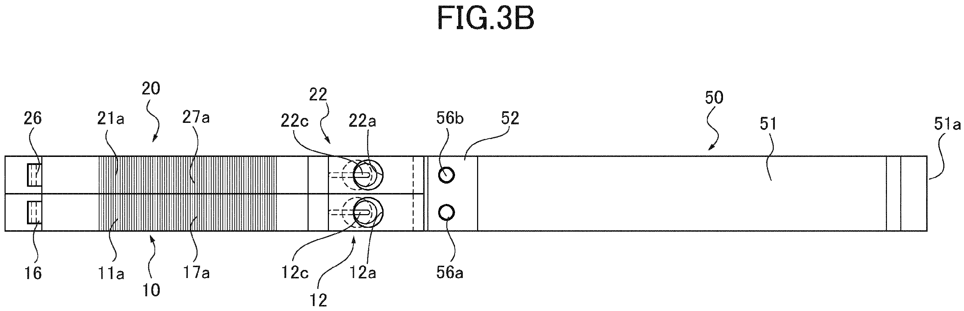

The drive member 50 is configured to collectively hold the first and second blades 10, 20 of the spreader body 30 and used upon striking or driving the spreader body 30 into the cut section 2. As shown in FIG. 1, the drive member 50 includes a grip portion 51 to be held by the surgeon, and a receiving portion 52 which receives the engaged portions 13, 23 of the spreader body 30. As shown in FIGS. 1 and 3A, the width of the grip portion 51 is narrower than that of the receiving portion 52 and the drive member 50 is configured to be easy to grip or hold. As shown in FIG. 3B, the thickness of the drive member 50 in the height direction is constant from the tip to the base end, which increases the strength of the drive member 50.

The grip portion 51 includes a driven or struck portion 51a at an end surface thereof. The struck portion 51a is struck or hit by the plastic hammer or the like. The receiving portion 52 includes the receiving recess 53, the engagement protrusion 54, the engagement ridges 55a, 55b, and ball plungers 56a, 56b.

The receiving recess 53 is formed at the tip side of the receiving portion 52 to receive the engagement protrusions 131a, 131b, 231a, 231b of the first and second blades 10, 20. The engagement protrusion 54 is provided at the center of the receiving recess 53 and longitudinally extends therefrom to engage with the engagement recesses 134a, 134b, 234a, 234b of the first and second blades 10, 20. The engagement ridges 55a, 55b inwardly extend from the tip side of the receiving recess 53 to engage with the engagement grooves 132a, 132b, 232a, 232b of the first and second blades 10, 20.

With this configuration, the engagement protrusion 54 and the engagement ridges 55a, 55b support the engagement protrusions 131a, 131b, 231a, 231b received within the receiving recess 53 in the longitudinal direction and the width direction perpendicular thereto. Thereby, displacement, misalignment, and the like in the height, width, and longitudinal directions can be prevented, and accordingly the drive member 50 can collectively and stably hold the first and second blades 10, 20.

As shown in FIG. 3B, the ball plunger 56a, 56b are provided on one of the side surfaces of the receiving portion 52 and aligned in the height direction. The first ball plunger 56a elastically engages with the ball receiver 133 of the first blade 10. The second ball plunger 56b elastically engages with the ball receiver 233 of the second blade 20. Thereby, the stability of the first and second blades 10, 20 held by the drive member 50 can be further improved and unexpected disengagement and/or misalignment can be prevented more properly.

The materials for the elements of the spreader 100 are not particularly limited, and known materials may be used. For example, various metal materials such as aluminum and stainless steel may be preferably used.

Also, the material for the artificial bone, which is inserted into the cut section 2 enlarged by the spreader 100, is not particularly limited, and known materials may be used. For example, ceramic materials are preferable, and bioceramics such as alumina, zirconia, and calcium phosphate compounds and the like are more preferable. Calcium phosphate compounds are particularly preferable because of the excellent biocompatibility thereof. For example, calcium phosphate compounds include apatites such as hydroxyapatite, fluorine apatite, carbonate apatite, and/or dicalcium phosphate, tricalcium phosphate, tetra calcium phosphate, octacalcium phosphate, and the like, which may be used alone or in a mixture of two or more.

The procedure of the high tibial osteotomy (open wedge) using the spreader 100 of the first embodiment configured as above will be described with reference to FIG. 3A to FIG. 7.

First, the drive member 50 is attached to the spreader body 30. As shown in FIGS. 3A and 3B, the first and second blades 10, 20 in the closed state are arranged in parallel such that the first arms 11a, 21a are aligned adjacent to each other and the second arms 11b, 21b are aligned adjacent to each other. In this state, the receiving recess 53 of the receiving portion 52 of the drive member 50 receives the engaged portions 13, 23. At this time, the engaged portions 13, 23 are inserted into the receiving recess 53 in the height direction so that the engaged portions 13, 23 easily engage with the receiving recess 53. Thereby, unexpected disengagement and/or misalignment can be prevented and the drive member 50 and the first and second blades 10, 20 can be stably connected.

Next, the tip of the spreader body 30 (first and second blades 10, 20) is set into the cut section 2 formed in the bone 1 by osteotomy. Then, the surgeon holds the grip portion 51 of the drive member 50 and strikes or hits the struck portion 51a with the plastic hammer to gradually insert the spreader body 30 (more specifically, insert portion 15a, 15b, 25a, 25b of first and second blades 10, 20) into the cut section 2. It is preferable that this insert operation is performed under X-ray fluoroscopy, for example. In the case of the high tibial osteotomy, it is preferable that the insertion depth of the spreader body 30 is set such that the tip of the spreader body 30 reaches the vicinity of the outer cortex.

In this inserted state, the first and second blades 10, 20 are closed and the angle θ1 between the arms 11a and 11b and the angle θ2 between the arms 21a and 21b are the minimum value (five degrees in this embodiment). In other words, only inserting the spreader body 30 into the cut section 2 can widen the cut section 2 by the minimum angle.

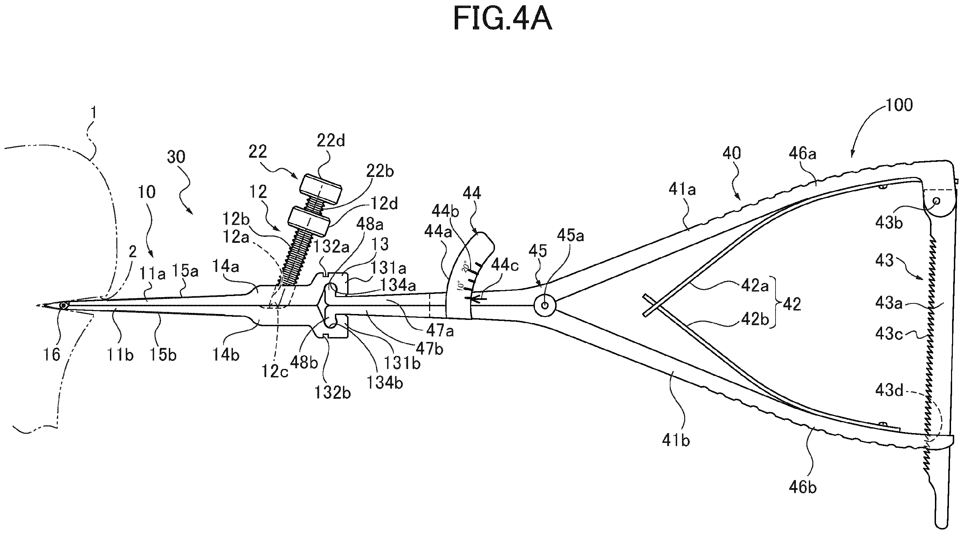

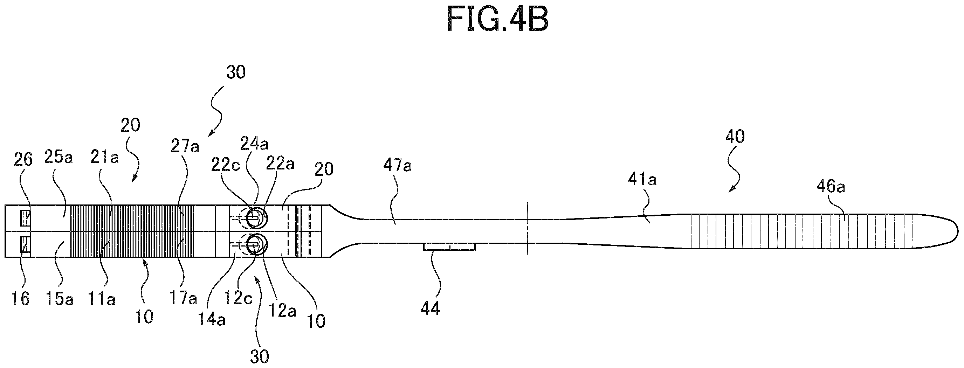

After completing the insertion of the cut section 2 of the spreader body 30, the drive member 50 is removed from the spreader body 30. Next, as shown in FIGS. 4A and 4B, the opening and closing mechanism 40 is attached to the spreader body 30 to enlarge or widen the cut section 2 by the spreader body 30. This attachment is performed by engaging the engagement protrusions 48a, 48b of the opening and closing mechanism 40 with the engagement recesses 134a, 134b, 234a, 234b of the engaged portions 13, 23 in the first and second blades 10, 20. Accordingly, the first and second blades 10, 20 can be collectively opened by the opening and closing mechanism 40.

Then, the surgeon holds the handles 46a, 46b of the opening and closing mechanism 40 to move the handles 46a, 46b in the directions close to each other. Thereby, the operation portions 47a, 47b rotate about the connection shaft 45a in the directions (opening direction) away from each other as shown in FIG. 5. At this time, the engagement protrusions 48a, 48b of the operation portions 47a, 47b engaged with the engagement recesses 134a, 134b, 234a, 234b push the arms 11a, 11b, 21a, 21b in the directions away from each other. Thereby, the arms 11a, 11b, 21a, 21b rotate about the connecting shafts 16a, 26a in the directions away from each other, and accordingly, the angles θ1, θ2 between the arms increase, which enlarges the cut section 2.

The angle scale member 44 of the opening and closing mechanism 40 indicates the angles θ1, θ2. The surgeon looks at the angle scale mark 44b pointed by the needle 44c of the angle scale member 44 and keeps performing the opening operation of the arms 11a, 11b, 21a, 21b with the handles 46a, 46b until the angles θ1, θ2 reach a target angle.

The first and second blades 10, 20 can be collectively opened only by the surgeon grabbing the handles 46a, 46b and moving the handles toward each other. Further, the angles θ1, θ2 between the arms 11a, 11b, 21a, 21b can be easily adjusted to the same angle at one time. Moreover, the gripping force applied to the handles 46a, 46b can be efficiently transmitted to the spreader body 30, and the operation can be effectively performed without excessive power or labor. Therefore, the cut section 2 can be easily and reliably enlarged to a predetermined size.

In addition, the ratchet mechanism 43 prevents the handles 46a, 46b from moving back in the separation directions even when the pressing force on the handles 46a, 46b is released. Therefore, the open states of the first and second blades 10, 20 can be maintained at the desired angles θ1, θ2.

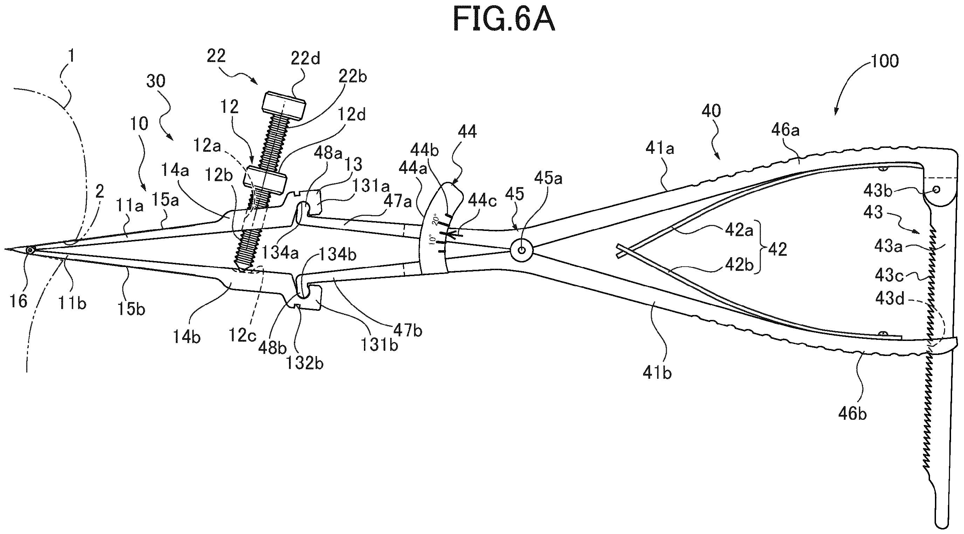

Next, the surgeon rotates the set screw 12b of the angle adjuster 12 in the first blade 10 in the fastening direction to make the tip of the set screw 12b contact with the inner surface of the recessed groove 12c of the second arm 11b as shown in FIG. 6A. Thereby, the open states of the arms 11a, 11b can be maintained at the desired angle θ1.

Then, the opening and closing mechanism 40 is removed from the spreader body 30. In this case, the opening and closing mechanism 40 slid in the height direction, or the lever 43a of the ratchet mechanism 43 is rotated to release the engagement of the ratchet teeth 43c and the teeth stopper 43d. Then, the biasing force of the biasing member 42 rotates the handles 46a, 46b in the separating directions to return the handles 46a, 46b to the original position. Thereby, the operation portions 47a, 47b are closed, and accordingly the angle between the operation portions 47a, 47b becomes smaller. Accordingly, the engagement of the engagement protrusions 48a, 48b and the engagement recesses 134a, 134b, 234a, 234b is released. As a result, the opening and closing mechanism 40 can be easily removed from the spreader body 30.

When the opening and closing mechanism 40 is removed from the spreader body 30, the arms 21a, 21b of the second blade 20 are closed. On the other hand, the open states of the arms 11a, 11b are maintained at the angle θ1 by the angle adjuster 12, and accordingly, the enlarged state of the cut section 2 is maintained.

Next, the second blade 20 is removed from the cut section 2 while the first blade 10 is kept inserted into the cut section 2. Since the arms 21a, 21b of the second blade 20 are closed, the second blade 20 can be easily removed from the cut section 2 as shown in FIG. 7.

The first blade 10 and the second blade 20 are not connected by protrusions or connecting members as in the prior art but are simply aligned in parallel adjacent to each other. Accordingly, there is no need to remove the connecting members or move them in the width direction as in the prior art. The second blade 20 can be easily removed by moving the second blade 20 along the longitudinal direction in the removing direction. It should be noted that the second blade 20 is first removed in this embodiment but the removing order is not limited thereto. The first blade 10 may be removed first. In this case, the first blade 10, in which the arms 11a, 11b are in the closed state after removing the opening and closing mechanism 40, is removed while the arms 21a, 21b of the second blade 20 are kept open by operating the angle adjuster 22.

Removing the second blade 20 as described above leaves space enough to insert the artificial bone into the cut section 2. In addition, the space does not unexpectedly decrease and the space with the desired size can be maintained since the first blade 10 is kept inserted into the cut section 2. Therefore, the insertion operation of the artificial bone into the space can be performed smoothly and quickly.

Next, the set screw 12b of the first blade 10 is rotated in a releasing or unfastening direction (removing direction from screw hole 12a) opposite to the fastening direction to remove the first blade 10 from the cut section 2. Then, the arms 11a, 11b of the first blade 10 are moved in the directions close to each other to decrease the angle θ1. When the angle θ1 becomes sufficiently small, the first blade 10 is easily removed from the cut section 2. Since the artificial bone has been inserted into the space after the removal of the second blade 20, the cut section 2 can be prevented from unexpectedly decreasing even when the arms 11a, 11b are closed and/or the first blade 10 is removed.

Also, the artificial bone or the like can be smoothly and quickly inserted into the space after the removal of the first blade 10. It should be noted that the two artificial bones are inserted into the cut section 2 in this embodiment. However, one artificial bone or three or more artificial bones may be inserted depending on the patient's body size or the like.

Next, predetermined procedures such as fixing the artificial bone inserted into the cut section 2 with titanium plates and/or bolts are performed to complete the high tibial osteotomy. The spreader 100 of this embodiment makes it possible to perform faster and more sophisticated operations.

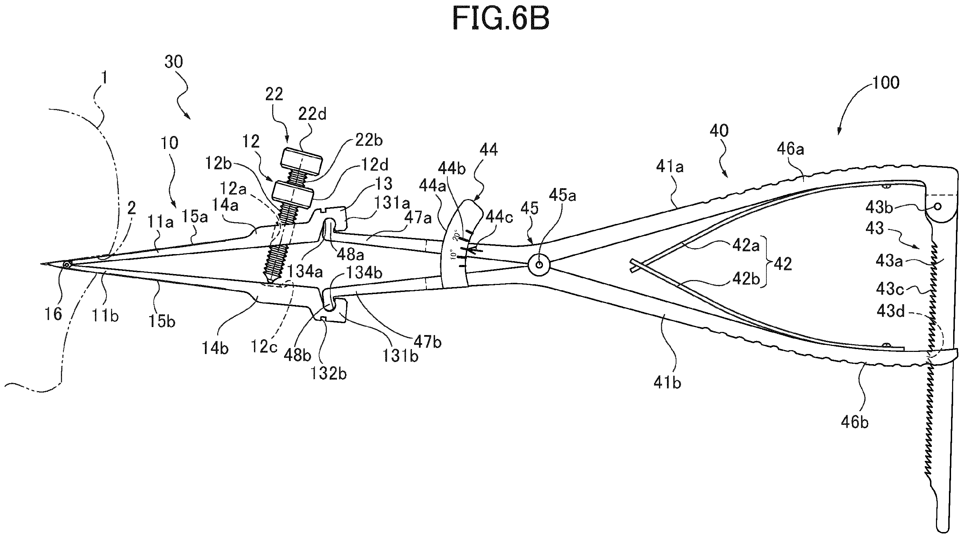

In the case described above, the angle adjuster 12 of the first blade 10 is operated to place the arms 11a, 11b in the open state before removing the opening and closing mechanism 40 from the spreader body 30. However, the present disclosure is not limited thereto. For example, as shown in FIG. 6B, the set screws 12b, 22b of the angle adjusters 12, 22 of the first and second blades 10, 20 may be rotated in the fastening direction to contact the tips of the set screws to the inner surfaces of the recessed grooves 12c, 22c of the second arms 11b, 21b. Thereby, the open states of the arms 11a, 11b, 21a, 21b can be maintained at the target angles θ1, θ2. Since the set screws 12b, 22b are arranged adjacent to each other, the surgeon or the like can rotate the set screws 12b, 22b from one side or direction and easily perform operations.

In this case, the open states of the arms 11a, 11b, 21a, 21b are maintained at the angles θ1, θ2 by the angle adjusters 12, 22. The enlarged state of the cut section 2 can be stably maintained after the opening and closing mechanism 40 is removed from the spreader body 30.

In the case that the second blade 20 is first removed from the cut section 2, for example, the set screw 22b of the second blade 20 is rotated in the unfastening direction. Thereby, the arms 21a, 21b of the second blade 20 are moved in the directions close to each other to decrease the angle θ2. Therefore, the second blade 20 can be easily removed from the cut section 2 similar to the example shown in FIG. 7.

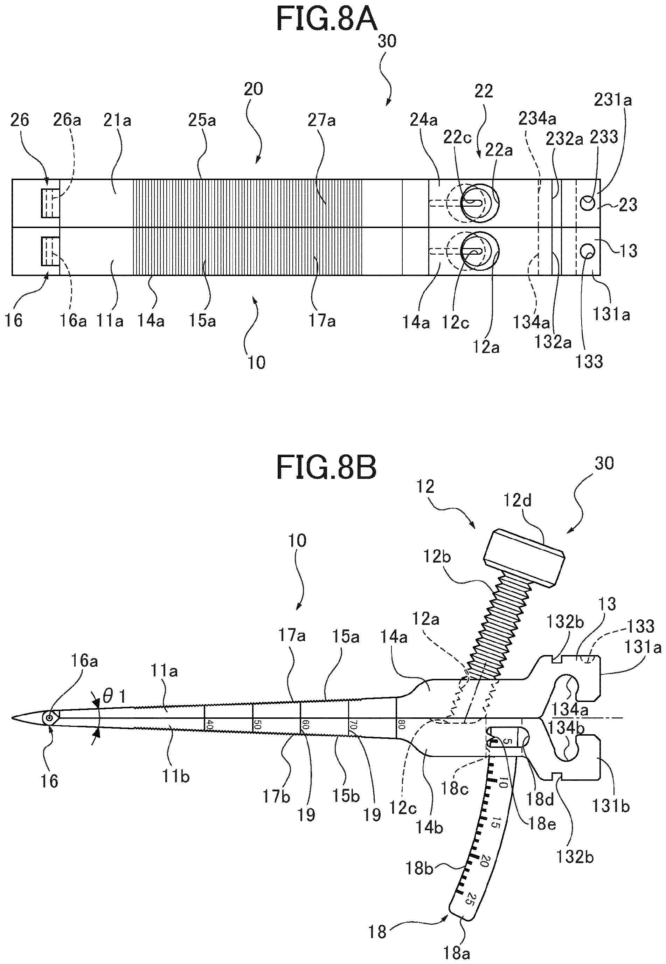

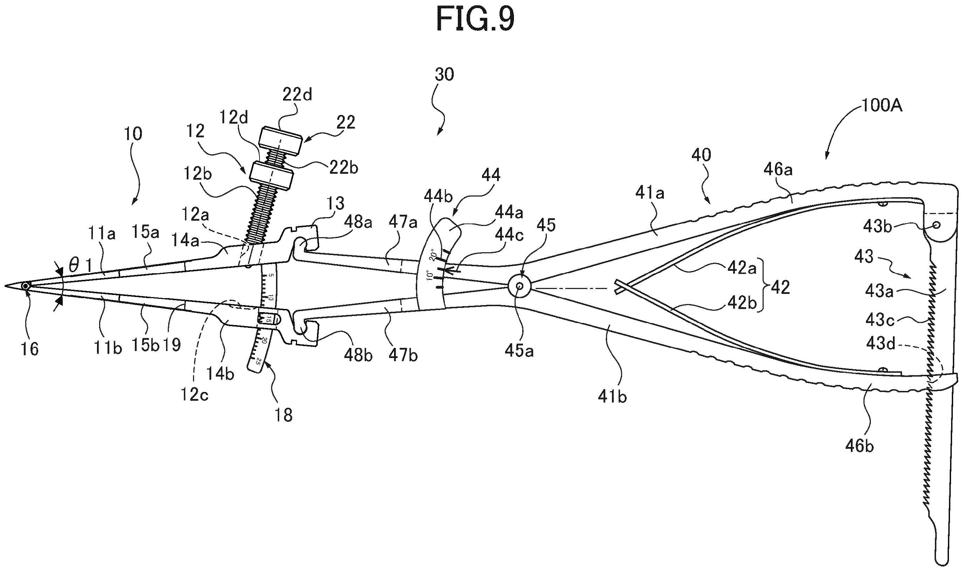

(Second Embodiment) Next, a spreader 100A of a second embodiment shown in FIGS. 8A, 8B, and 9 will be described. FIG. 8A is a side view illustrating the first and second blades 10, 20. FIG. 8B is a plan view illustrating the first blade 10. FIG. 9 is a plan view illustrating the open state of the spreader body 30 to which the opening and closing mechanism 40 is attached.

The spreader 100A of the second embodiment has a basic configuration similar to that of the spreader 100 of the first embodiment shown in FIGS. 1 to 7 except that the first blade 10 is provided with an angle scale member (second angle scale member) 18 and an insertion depth scale 19. Also, the spreader 100A of the second embodiment is operated similarly to the spreader 100 of the first embodiment. Therefore, the elements similar to those in the first embodiment are denoted with the same reference numerals as in the first embodiment, and the detailed descriptions of the configurations and operations similar to those in the first embodiment are omitted. Hereinafter, features such as configurations different from those of the first embodiment will be mainly described.

As shown in FIGS. 8A to 9, the spreader 100A of the second embodiment includes the spreader body 30 and the opening and closing mechanism 40. The spreader body 30 includes the first blade 10 and the second blade 20. Also in the second embodiment, the drive member 50 such as one shown in FIG. 1 or the like is provided. The second blade 20, the opening and closing mechanism 40, and the drive member 50 in this embodiment may be similar to or the same as those in the first embodiment.

Similar to the first blade 10 of the first embodiment, the first blade 10 of the second embodiment includes the arms 11a, 11b, the angle adjuster 12, and the engaged portion 13. The arms 11a, 11b include the base end portion 14a, 14b, and the insert portion 15a, 15b, respectively. Further, the first blade 10 includes the angle scale member 18 and the insertion depth scale 19.

The second blade 20 has a configuration similar to that of the second blade 20 of the first embodiment. It should be noted that the second blade 20 may include the angle scale member and/or the insertion depth scale instead of the first blade 10. Alternatively, both of the first and second blades 10, 20 may include the angle scale member and/or the insertion depth scale. In this case, there is no need to distinguish the first and second blades 10, 20. Also, the first and second blades 10, 20 may be manufactured in the same process using the same mold or die, etc., which improves productivity thereof.

As shown in FIG. 8B, the second angle scale member 18 includes a plate 18a, an angle scale marks 18b, an insertion hole 18c, a window 18d, and a needle 18e. The angle scale marks 18b are provided on the front surface of the plate 18a. The insertion hole 18c removably receives the plate 18a. The angle scale marks 18b of the plate 18a within the insertion hole 18c can be seen through the window 18d. The needle 18e points to one of the angle scale marks 18b.

One end of the plate 18a is fixed to the inner surface of the base end portion 14a of the first arm 11a. The plate 18a has an arc shape with the connection shaft 16a as a center and extends from the inner surface to the second arm 11b.

The insertion hole 18c is provided in the base end portion 14b of the second arm 11b to extend through the base end portion 14b in the extending direction of the plate 18a between the inner surface and the outer surface thereof. The window 18d has an oval shape in the longitudinal direction and is provided on the front side of the base end portion 14b to communicate with the insertion hole 18c. The needle 18e is provided by inwardly extending a part of the inner surface of the window 18d to point to one of the angle scale marks 18b.

In the angle scale member 18 configured as above, when the arms 11a, 11b of the first blade 10 rotationally moves, the plate 18a smoothly moves within the insertion hole 18c. The angle θ1 between the arms 11a, 11b can be grasped easily and accurately by reading the angle scale mark 18b, which is pointed by the needle 18e, through the window 18d.

The insertion depth scale 19 is configured to indicate the insertion depth (depth) of the insert portion 15a, 15b of the arms 11a, 11b of the first blade 10 inserted into the cut section 2 of the bone 1. As shown in FIG. 8B, the insertion depth scale 19 is provided on the front surfaces of the insert portion 15a, 15b in the longitudinal direction.

Similar to the first embodiment, in the spreader 100A of the second embodiment configured as above, the first and second blades 10, 20 are stably and collectively held by the drive member 50 so that the insertion operation into the cut section 2 can be easily performed. Further, the insertion depth scale 19 makes it possible for the surgeon or the like to easily and clearly know the insertion depth of the spreader body 30 into the cut section 2 without a separate gauge when the spreader body 30 is inserted into the cut section 2.

In addition, as shown in FIG. 9, the surgeon operates the opening and closing mechanism 40 attached to the spreader body 30 to open the first and second blades 10, 20 at one time. At this time, the first angle scale member 44 of the opening and closing mechanism 40 makes it possible for the surgeon or the like to know the angles between the operation portions 47a, 47b, that is the angles θ1, θ2 between the arms 11a, 11b, 21a, 21b. Moreover, the second angle scale member 18 provided in the first blade 10 also makes it possible for the surgeon or the like to know the angle θ1 between the arms 11a, 11b.

Further, the second angle scale member 18 makes it possible for the surgeon or the like to know the angle θ1 between the arms 11a, 11b even when the opening and closing mechanism 40 is removed from the spreader body 30 or the opening and closing mechanism 40 does not include the first angle scale member 44.

The angle scale member 18 is inserted into the insertion hole 18c of the base end portions 14a, 14b (or base end portions 24a, 24b when it is provided in second blade 20). Accordingly, the first and second blades 10, 20 have no irregularities on the front and back surfaces so that the first and second blades 10, 20 can be aligned in parallel adjacent to each other from the front or back surface side. Therefore, the first and second blades 10, 20 can be arranged more freely.

In the invention disclosed in Patent Literature V, for example, the surgeon or the like has to change his or her posture when checking the scales since the angle scale (angle scale 61) and the insertion depth scale (scale 26) are provided on the different surfaces that intersect each other. In addition, the position of the angle scale marks may be misaligned since the member (connection member 6) including the angle scale marks is attached to the blade such that the scale can be inserted into and removed from the blade.

On the other hand, in the second embodiment, the first angle scale member 44, the second angle scale member 18, and the insertion depth scale 19 are arranged on the substantially same plane (front surface side) when the opening and closing mechanism 40 is attached to the spreader body 30. The phrase "arranged on the substantially same plane" herein does not necessarily mean that the above members are provided on the same plane but means that the above members are provided to be arranged on parallel planes that can be seen from the same direction. Thereby, the surgeon or the like can easily see these scale marks from the same direction without changing his or her posture, and accordingly, the operations can be performed smoothly and quickly. In addition, one end of the plate 44a of the first angle scale member 44 is fixed to the shaft 41b and one end of the plate 18a of the second angle scale member 18 is fixed to the base end portion 14a. Thereby, the displacement of the scale marks can be prevented, and accordingly, the angles can be always measured accurately.

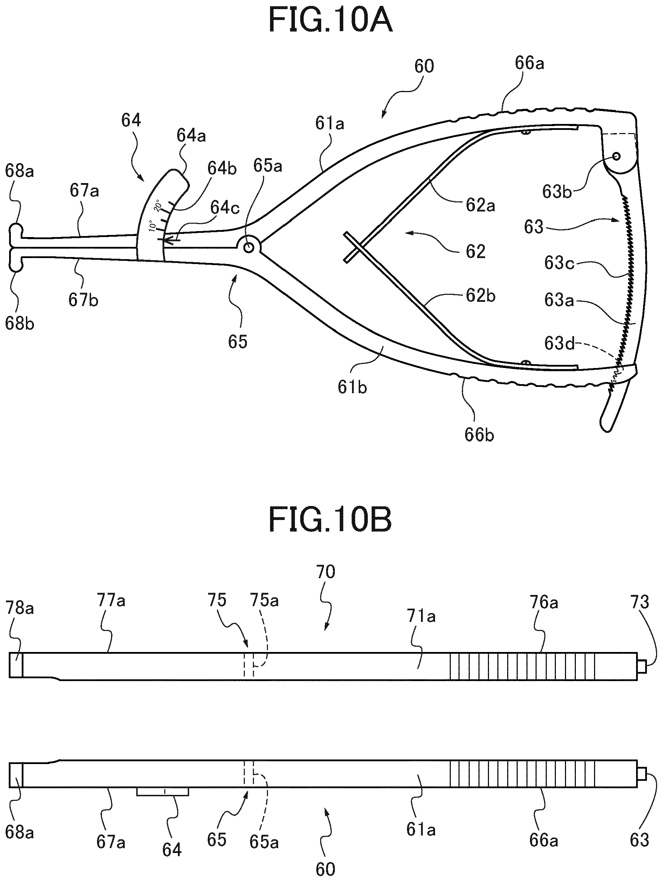

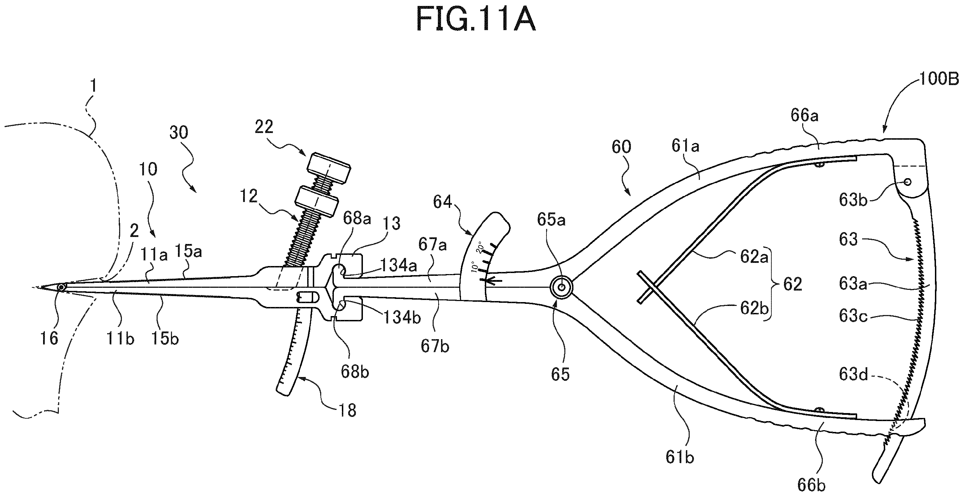

(Third Embodiment) Next, a spreader according to a third embodiment will be described with reference to FIG. 10A to FIG. 12. As shown in FIGS. 11A to 12, the spreader 100B of the third embodiment includes a first opening and closing mechanism 60 and a second opening and closing mechanism 70 which are opened and closed independently. The spreader 100B of the third embodiment has a basic configuration substantially the same as that of the spreader 100A of the second embodiment shown in FIGS. 8A to 9 except that the spreader 100B includes the first and second opening and closing mechanisms 60, 70 instead of the opening and closing mechanism 40. Hereinafter, the configurations different from those of the second embodiment will be mainly described.

The spreader 100B of the third embodiment includes the spreader body 30 including the first and second blades 10, 20, the first opening and closing mechanism 60, the second opening and closing mechanism 70, and the drive member 50 (see FIG. 1). In the third embodiment and fourth to ninth embodiments, which will be described later, the first and second blades similar to the ones in the second embodiment are used as the first and second blades 10, 20 (spreader body 30). However, the first and second blades 10, 20 similar to the ones in the first embodiment may be used. Also, in the third to ninth embodiments, a drive member similar to the one in the first embodiment may be used as the drive member 50.

Hereinafter, the first and second opening and closing mechanisms 60, 70 will be described in detail. The first opening and closing mechanism 60 is arranged in parallel with the second opening and closing mechanism 70 adjacent to each other so that the first and second blades 10, 20 of the spreader body 30 are opened and closed collectively or individually.

As shown in FIGS. 10A to 12, the first opening and closing mechanism 60 includes a pair of shafts 61a, 61b, a biasing member 62, a ratchet mechanism 63, and a first angle scale member 64. The first angle scale member may be provided in the second opening and closing mechanism 70 or both of the first and second opening and closing mechanisms 60, 70. The shafts 61a, 61b are rotatably connected by a connection portion 65. For example, the connection portion 65 has a pivot structure including a connection shaft 65a and a bearing. The shafts 61a, 61b respectively include handles 66a, 66b at the base end side and the operation portions 67a, 67b at the tip side thereof with the connection shaft 65a located therebetween. The operation portions 67a, 67b are opened and closed in conjunction with the opening and closing movements of the handles 66a, 66b.

The operation portions 67a, 67b have an L-shape in a plan view. Specifically, the operation portions 67a, 67b respectively include the engagement protrusions 68a, 68b at the respective tips thereof that extend in the width direction perpendicular to the longitudinal direction. For example, the first engagement protrusion 68a engages with the first engagement recess 134a of the first blade 10 while the second engagement protrusion 68b engages with the second engagement recess 134b of the first blade 10.

Similarly, the second opening and closing mechanism 70 is arranged in parallel with the first opening and closing mechanism 60 adjacent to each other so that the first and second blades 10, 20 of the spreader body 30 are opened and closed collectively or individually. The second opening and closing mechanism 70 includes a pair of shafts 71a, 71b, a biasing member 72, and a ratchet mechanism 73. The shafts 71a, 71b are rotatably connected with a connection portion 75. For example, the connection portion 75 has a pivot structure including a connection shaft 75a and a bearing. The shafts 71a, 71b respectively include handles 76a, 76b at the base end side and operation portions 77a, 77b at the tip side with the connection shaft 75a located therebetween. The operation portions 77a, 77b are opened and closed in conjunction with the opening and closing movements of the handles 76a, 76b.

Each of the operation portions 77a, 77b has an L-shape in a plan view. Specifically, the operation portions 77a, 77b respectively include the engagement protrusions 78a, 78b at the respective tips thereof that extend in the width direction perpendicular to the longitudinal direction. For example, the first engagement protrusion 78a engages with the first engagement recess 234a of the second blade 20 while the second engagement protrusion 78b engages with the second engagement recess 234b of the second blade 20.

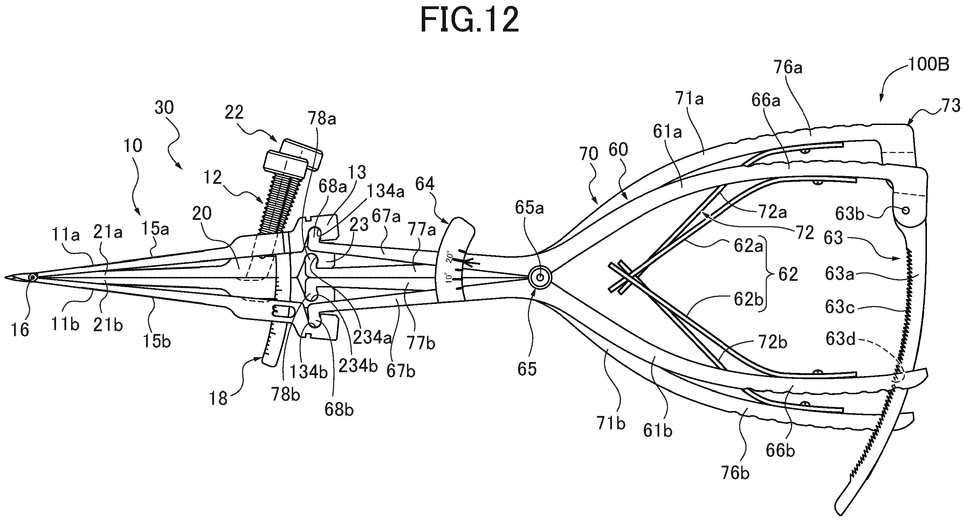

FIG. 11A shows the first blade 10 that is inserted into the cut section 2 and to which the first opening and closing mechanism 60 is attached. The second opening and closing mechanism 70 is attached to the second blade 20. In the combination shown in FIG. 11A, the engagement protrusion 68a of the first operation portion 67a of the first opening and closing mechanism 60 engages with the first engagement recess 134a of the first blade 10. The engagement protrusion 68b of the second operation portion 67b engages with the second engagement recess 134b of the first blade 10. Also, the engagement protrusion 78a of the first operation portion 77a of the second opening and closing mechanism 70 engages with the first engagement recess 234a of the second blade 20. The engagement protrusion 78b of the second operation portion 77b engages with the second engagement recess 234b of the second blade 20 (see FIG. 12 for operation portions 77a, 77b, engagement protrusions 78a, 78b, and engagement recesses 234a, 234b).

The combination of the first and second blades 10, 20 of the spreader body 30 and the first and second opening and closing mechanisms 60, 70 may be arbitrarily selected depending on an applied part (bone 1) of the patient (i.e., left or right side of patient) by considering the orientations of the first angle scale member 64 and the second angle scale member 18, or the like. Therefore, the second opening and closing mechanism 70 may be attached to the first blade 10 and the first opening and closing mechanism 60 may be attached to the second blade 20.

Similar to the first and second embodiments, the first angle scale member 64 includes a plate 64a, an angle scale 64b, and a needle 64c. The ratchet mechanism 63 includes a lever 63a, a connection shaft 63, and a teeth stopper 63d. The lever 63a includes a plurality of ratchet teeth 63c. The closing operation of the handles 66a, 66b for one of the ratchet teeth 63c opens the operation portions 67a, 67b at an angle of 0.5 degrees. The ratchet mechanism 73 has a configuration similar to the ratchet mechanism 63 and the operation portions 77a, 77b also are opened at an angle of 0.5 degrees.

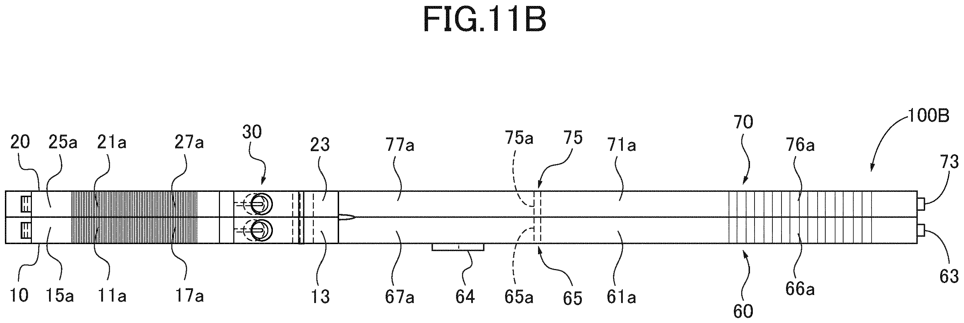

The surgeon holds the handles 66a, 66b of the first opening and closing mechanism 60 for example, and moves the handles in the directions close to each other from the closed state of the first and second opening and closing mechanisms 60, 70 shown in FIGS. 11A, 11B. This operation rotates the operation portions 67a, 67b about the connection shaft 65a in the directions away from each other as shown in FIG. 12, which moves the arms 11a, 11b of the first blade 10 in the directions away from each other. Thereby, the first blade 10 is opened at a predetermined angle (θ1).

Next, the arms 21a, 21b of the second blade 20 are opened at a predetermined angle (θ2) by operating the handles 76a, 76b of the second opening and closing mechanism 70. In this way, in the third embodiment, the first and second blades 10, 20 can be opened individually by operating the first and second opening and closing mechanisms 60, 70 individually. In addition, the arms 11a, 11b of the first blade 10 and the arms 21a, 21b of the second blade 20 may be opened by different angles θ1, θ2.

Also in the third embodiment, the arms 11a, 11b, 21a, 21b of the first and second blades 10, 20 can be collectively opened at the same angle θ1, θ2 by collectively operating the first and second opening and closing mechanisms 60, 70. The surgeon can freely select the collective operation or the individual operation according to the surgical site, surgical field space, or the like.

On the other hand, when the first and second opening and closing mechanisms 60, 70 are removed from the first and second blades 10, 20, the first and second opening and closing mechanisms 60, 70 are slid in the height direction or the ratchet mechanisms 63, 73 are returned to predetermined positions similar to the opening and closing mechanism 40 in the first embodiment. Thereby, the first and second opening and closing mechanisms 60, 70 can be removed collectively or individually.

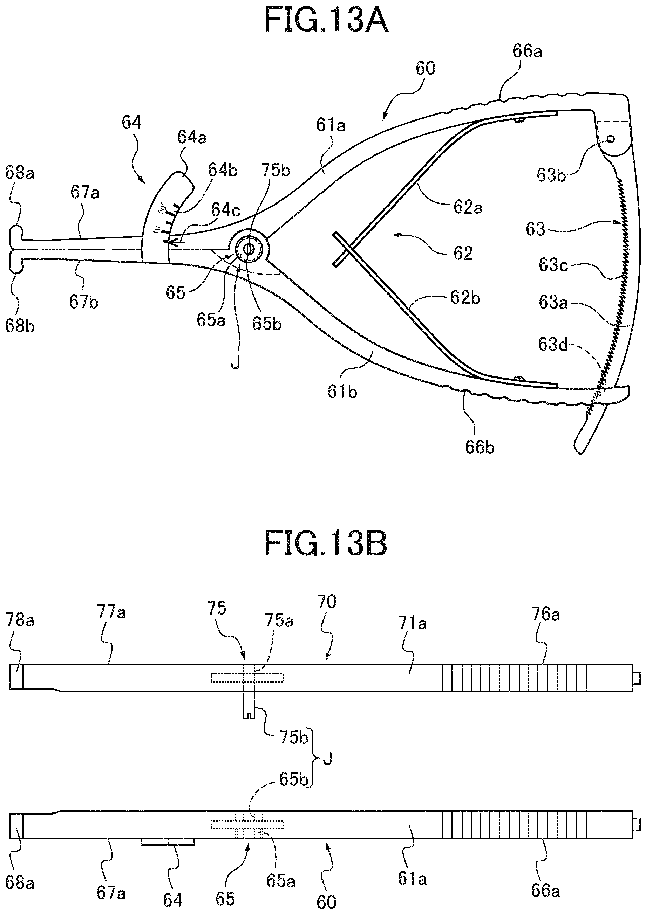

(Fourth Embodiment) Next, a spreader according to a fourth embodiment will be described with reference to FIGS. 13A and 13B. As shown in FIGS. 13A and 13B, the spreader of the fourth embodiment has a basic configuration similar to the spreader 100B of the third embodiment shown in FIGS. 10A to 12 except that the first and second opening and closing mechanisms 60, 70 include an engagement mechanism J that connects and removably engages the opening and closing mechanisms 60, 70 with respect to each other. Specifically, the spreader of the fourth embodiment includes the spreader body 30 including the first and second blades 10, 20 (see FIGS. 8A to 9), the opening and closing mechanisms 60, 70, and the drive member 50 (see FIG. 1).

Hereinafter, the configuration of the engagement mechanism J, which is different from the third embodiment, will be mainly described. The shafts 61a, 61b of the first opening and closing mechanism 60 are rotatably connected by the connection portion 65 which includes the connection shaft 65a and the bearing. Similarly, the shafts 71a, 71b of the second opening and closing mechanism 70 are rotatably connected by the connection portion 75 which includes the connection shaft 75a and the bearing. The connection shaft 75a of the second opening and closing mechanism 70 is integrally provided with an engagement protrusion 75b which outwardly extends toward the first opening and closing mechanism 60. The connection shaft 65a of the first opening and closing mechanism 60 is made of a cylindrical member having a diameter larger than that of the connection shaft 75a. This cylindrical member includes an engagement recess 65b that engages with the engagement protrusion 75b.

The engagement mechanism J includes the engagement recess 65b and the engagement protrusion 75b. In the fourth embodiment, the first opening and closing mechanism 60 includes the engagement recess 65b, and the second opening and closing mechanism 70 includes the engagement protrusion 75b. However, the present disclosure is not limited to the above configurations. The first opening and closing mechanism 60 may include the engagement protrusion and the second opening and closing mechanism 70 may include the engagement recess. This applies to other embodiments including the engagement mechanism J.

In the fourth embodiment with the above configurations, the connection shafts 65a, 75a connect the first and second opening and closing mechanisms 60, 70 by engaging the engagement protrusion 75b with the engagement recess 65b. Accordingly, positioning when the first and second opening and closing mechanisms 60, 70 are arranged in parallel adjacent to each other can be performed more easily and accurately. In addition, the first and second opening and closing mechanisms 60, 70 can be more effectively and stably attached to the first and second blades 10, 20.

Further, the first and second opening and closing mechanisms 60, 70 engage with each other by the connection shafts 65a, 75a. Accordingly, the movements of the shafts 61a, 61b, 71a, 71b are not interfered, so that the shafts can move independently. Moreover, the stability when the surgeon holds the mechanisms 60, 70, and performs the operations can be improved. Similar to the third embodiment, in the spreader of the fourth embodiment, the first and second blades 10, 20 can be collectively or individually opened and closed at a predetermined angle or angles by collectively or individually operating the first and second opening and closing mechanisms 60, 70. Moreover, the first and second opening and closing mechanisms 60, 70 can be easily disconnected by disengaging the engagement protrusion 75b and the engagement recess 65b, which allows the first and second opening and closing mechanisms 60, 70 to be stored compactly.

In the fourth embodiment, the first and second opening and closing mechanisms 60, 70 include the engagement mechanism J which connects and removably engages the mechanisms 60, 70. In another embodiment, the first and second opening and closing mechanisms 60, 70 may be inseparably connected by a connection mechanism. For example, in such a connection mechanism, the engagement protrusion 75b and the engagement recess 65b may be inseparably connected by calking, swaging, or the like. With this configuration, the connection of the engagement protrusion 75b and the engagement recess 65b can eliminate the need for positioning to arrange the first and second opening and closing mechanisms 60, 70 in parallel. Further, the first and second opening and closing mechanisms 60, 70 can be prevented from accidentally falling even if the surgeon releases his or her hand from one of the opening and closing mechanisms 60, 70, which improves stability during the operation and makes them easier to carry. Also in this embodiment, the first and second opening and closing mechanisms 60, 70 can be collectively or individually operated, and accordingly, the first and second blades 10, 20 can be collectively or individually opened and closed at a predetermined angle or angles.

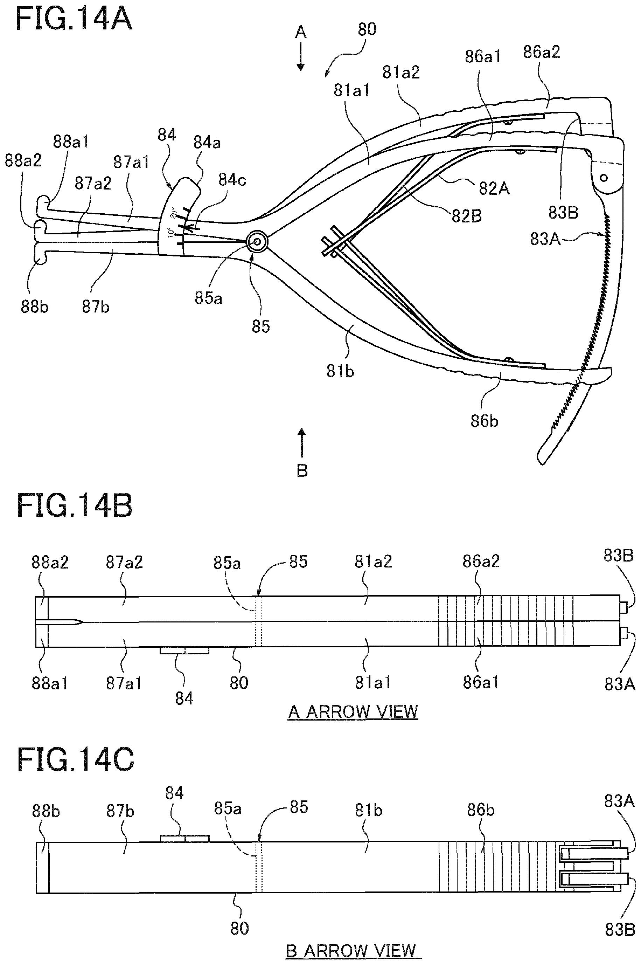

(Fifth Embodiment) Next, a spreader according to a fifth embodiment will be described with reference to FIGS. 14A to 14C. As shown in FIGS. 14A to 14C, the spreader of the fifth embodiment has a basic configuration similar to the spreader 100A of the second embodiment shown in FIGS. 8A to 9 except that an opening and closing mechanism 80 shown in FIGS. 14A to 14C is provided instead of the opening and closing mechanism 40. Specifically, the spreader of the fifth embodiment includes the spreader body 30 (see FIGS. 8A to 9) including the first blade 10 and the second blade 20, the opening and closing mechanism 80, and the drive member 50 (see FIG. 1).

Hereinafter, the configuration of the opening and closing mechanism 80 will be described in detail. The opening and closing mechanism 80 includes a first operation shaft 81a1 and a second operation shaft 81a2, a support shaft 81b, a first biasing member 82A, and a second biasing member 82B, a first ratchet mechanism 83A, and a second ratchet mechanism 83B, and a first angle scale member 84. The first and second operation shafts 81a1, 81a2 are arranged parallel to each other. The support shaft 81b is rotatably connected to the first and second operation shafts 81a1, 81a2 via a connection portion 85. The first biasing member 82A and the first ratchet mechanism 83A are provided for the first operation shaft 81a1. The second biasing member 82B and the second ratchet mechanism 83B are provided for the second operation shaft 81a2. The first angle scale member 84 is disposed in the support shaft 81b and includes a plate 84a, angle scale marks 84b, and a needle 84c. For example, the connection portion 85 has a pivot structure and includes a connection shaft 85a and a bearing.

The first operation shaft 81a1 includes a first handle 86a1 at the base end side thereof and the second operation shaft 81a2 includes a second handle 86a2 at the base end side thereof. The first and second handles 86a1, 86a2 respectively include operation portions 87a1, 87a2 at the tip side thereof. The connection shaft 85a is located between the handles 86a1, 86a2 and the operation portions 87a1,87a2. The operation portions 87a1, 87a2 are opened and closed in conjunction with the opening and closing movements of the first and second handles 86a1, 86a2.

Each of the first and second operation portions 87a1, 87a2 has an L-shape in a plan view. Specifically, the first and second operation portions 87a1, 87a2 respectively include, at the respective tips thereof, the first and second engagement protrusions 88a1, 88a2 that extend in the width direction perpendicular to the longitudinal direction. The first engagement protrusion 88a1 engages with the engagement recess 134a of the first blade 10, for example. The second engagement protrusion 88a2 engages with the engagement recess 234a of the second blade 20, for example.

One support shaft 81b is provided for the two first and second operation shafts 81a1, 81a2. The thickness of the support shaft 81b in the height direction is substantially the same as the total thickness of the two operation shafts 81a1, 81a2. The support shaft 81b includes the handle 86b at the base end side and an operation portion 87b at the tip side with the connection shaft 85a located therebetween. The operation portion 87b is opened and closed in conjunction with the opening and closing movement of the handle 86b.

The operation portion 87b of the support shaft 81b has an L-shape in a plan view. Specifically, the operation portion 87b includes an engagement protrusion 88b at the tip thereof that extends in the width direction perpendicular to the longitudinal direction. For example, the engagement protrusion 88b engages with the second engagement recess 134b of the first blade 10 and the second engagement recess 234b of the second blade 20.

The combination of the first and second blades 10, 20 and the opening and closing mechanism 80 may be arbitrarily selected depending on an applied part (bone 1) of the patient (i.e., left or right side of patient) by considering the orientations of the first angle scale member 84, the second angle scale member 18, or the like. Therefore, the first and second blades 10, 20 and the opening and closing mechanism 80 may be combined such that the first engagement protrusion 88a1 of the first operation shaft 81a1 engages with the first engagement recess 234a of the second blade 20 while the second engagement protrusion 88a2 of second operation shaft 81a2 engages with the first engagement recess 134a of the first blade 10.

Alternatively, the first and second blades 10, 20 and the opening and closing mechanism 80 may be combined such that the first and second engagement protrusions 88a1, 88a2 of the first and second operation shafts 81a1, 81a2 engage with any of the second engagement recesses 134b, 234b of the first and second blades 10, 20 while the engagement protrusion 88b of the support shaft 81b engages with the first engagement recesses 134a, 234a of the first and second blades 10, 20.

In the fifth embodiment, the first and second blades 10, 20 of the spreader body 30 can be collectively or individually opened and closed at predetermined angles by collectively or individually operating the first operation shaft 81a1 or the second operation shaft 81a2 relative to the support shaft 81b.

More specifically, when the first blade 10 is opened first, for example, the surgeon holds the handle 86b of the support shaft 81b and the first handle 86a1 of the first operation shaft 81a1 and moves these handles in the directions close to each other. This operation rotates the first operation portion 87a1 in the direction away from the operation portion 87b of the support shaft 81b as shown in FIG. 14A. Thereby, the arms 11a, 11b of the first blade 10 rotate in the directions away from each other and open at a predetermined angle (θ1).

Next, when the second blade 20 is opened, the surgeon holds the handle 86b of the support shaft 81b and the second handle 86a2 of the second operation shaft 81a2 and moves the handles in the directions close to each other. This operation rotates the second operation portion 87a2 in the direction away from the operation portion 87b of the support shaft 81b. Thereby, the arms 21a, 21b of the second blade 20 rotate in the directions away from each other and open at a predetermined angle (θ2).

In this way, in the fifth embodiment, the first and second blades 10, 20 can be opened individually by individually operating the first and second operation shafts 81a1, 81a2 relative to the support shaft 81b. Further, the arms 11a, 11b of the first blade 10 and the arms 21a, 21b of the second blade 20 can be opened at different angles θ1, θ2.

Also in the fifth embodiment, the arms 11a, 11b, 21a, 21b of the first and second blades 10, 20 can be collectively opened at the same angle θ1, θ2 by collectively operating the first and second operation shafts 81a1, 81a2 relative to the support shaft 81b. The surgeon can freely select the collective operation or the individual operation according to the surgical site, surgical field space, or the like.

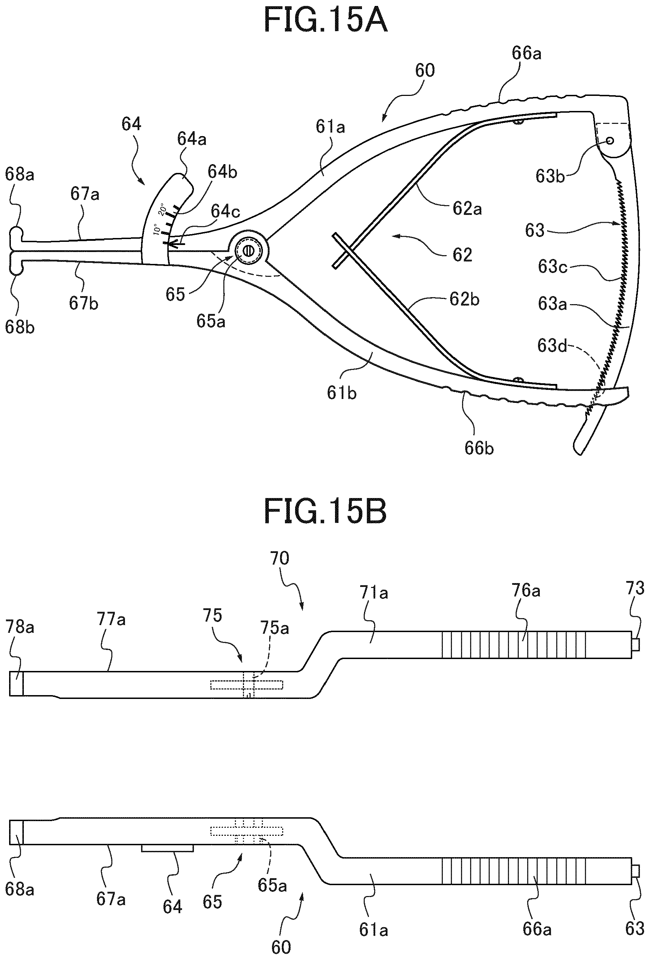

(Sixth Embodiment) Next, a spreader according to a sixth embodiment will be described with reference to FIGS. 15A and 15B. As shown in FIGS. 15A and 15B, the spreader of the sixth embodiment has a basic configuration similar to that of the spreader 100B of the third embodiment shown in FIGS. 11A to 12 except that the shapes of the shafts 61a, 61b, 71a, 71b of the first and second opening and closing mechanisms 60, 70 are different from those of the shafts in the third embodiment. According to the sixth embodiment, the same effect as the third embodiment can be obtained. Therefore, the configuration of the spreader and effect of the sixth embodiment will not be described in detail.

In the sixth embodiment, the handles 66a, 66b of the shafts 61a, 61b of the first opening and closing mechanism 60, and the handles 76a, 76b of the shafts 71a, 71b of the second opening and closing mechanism 70 are curved or bent to protrude in the respective directions away from the each other as shown in FIG. 15B. In the other words, the handles 66a, 66b, 76a, 76b of the shafts 61a, 61b, 71a, 71b offset with respect to the operation portions 67a, 67b, 77a, 77b (also referred to as "offset handle" hereinafter). According to the above structures, the handles 66a, 66b of the first opening and closing mechanism 60 and the handles 76a, 76b of the second opening and closing mechanism 70 are arranged away from each other with a predetermined distance therebetween when the operation portions 67a, 67b, 77a, 77b are arranged in parallel adjacent to each other.

Similar to the third embodiment, in the sixth embodiment as configured above, the first and second blades 10, 20 can be collectively or individually opened and closed at a predetermined angle or angles by collectively or individually operating the first and second opening and closing mechanisms 60, 70. Further, in the sixth embodiment, the handles 66a, 66b, 76a, 76b offset from each other. This structure produces a significant effect that makes it easier to secure the field of view and the space of the surgical field on the straight line from the affected part (bone 1) when the first and second blades 10, 20 are opened by the first and second opening and closing mechanisms 60, 70 to enlarge the affected part.

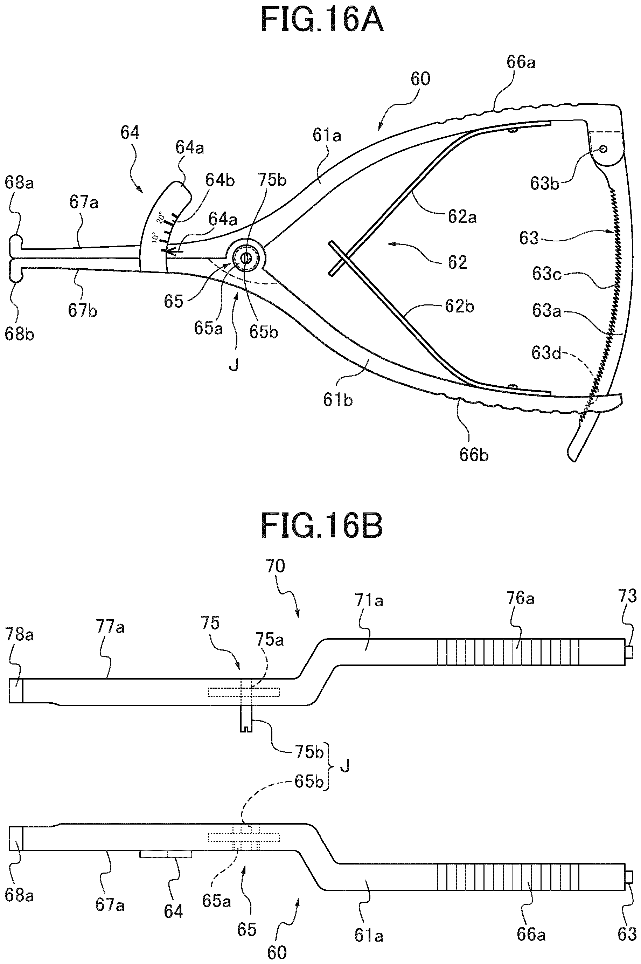

(Seventh Embodiment) Next, a spreader according to a seventh embodiment will be described with reference to FIGS. 16A and 16B. As shown in FIGS. 16A and 16B, the spreader of the seventh embodiment has a basic configuration similar to that of the spreader of the sixth embodiment including the first and second opening and closing mechanisms 60, 70, which are shown in FIGS. 15A and 15B except that the first and second opening and closing mechanisms 60, 70 are provided with the engagement mechanism J that connects the mechanisms 60, 70 and engages them with each other. Similar to the fourth embodiment, the engagement mechanism J in the seventh embodiment includes the engagement protrusion 75b and the engagement recess 65b. The engagement protrusion 75b is provided on the connection shaft 75a of the second opening and closing mechanism 70 to extend therefrom. The engagement recess 65b is provided within the cylindrical connection shaft 65a of the first opening and closing mechanism 60. It should be noted that the first and second opening and closing mechanisms 60, 70 may be inseparably connected by an appropriate connection mechanism in the seventh embodiment.

In the spreader of the seventh embodiment configured as above, the engagement of the engagement protrusion 75b and the engagement recess 65b can easily and accurately position the first and second opening and closing mechanisms 60, 70 when arranging the mechanisms 60, 70 in parallel adjacent to each other. Thereby, the first and second opening and closing mechanisms 60, 70 can be efficiently and securely attached to the first and second blades 10, 20. Also, the stability of the operation of the first and second opening and closing mechanisms 60, 70 by the surgeon can be improved. Moreover, the first and second opening and closing mechanisms 60, 70 can be easily separated and compactly stored by disengaging the engagement protrusion 75b and the engagement recess 65b.

The first and second blades 10, 20 can be collectively or individually opened and closed at a predetermined angle or angles by collectively or individually operating the positioned first and second opening and closing mechanisms 60, 70. In addition, offsetting the handles 66a, 66b, 76a, 76b makes it easier to secure the field of view and the space of the surgical field on the straight line from the affected part.

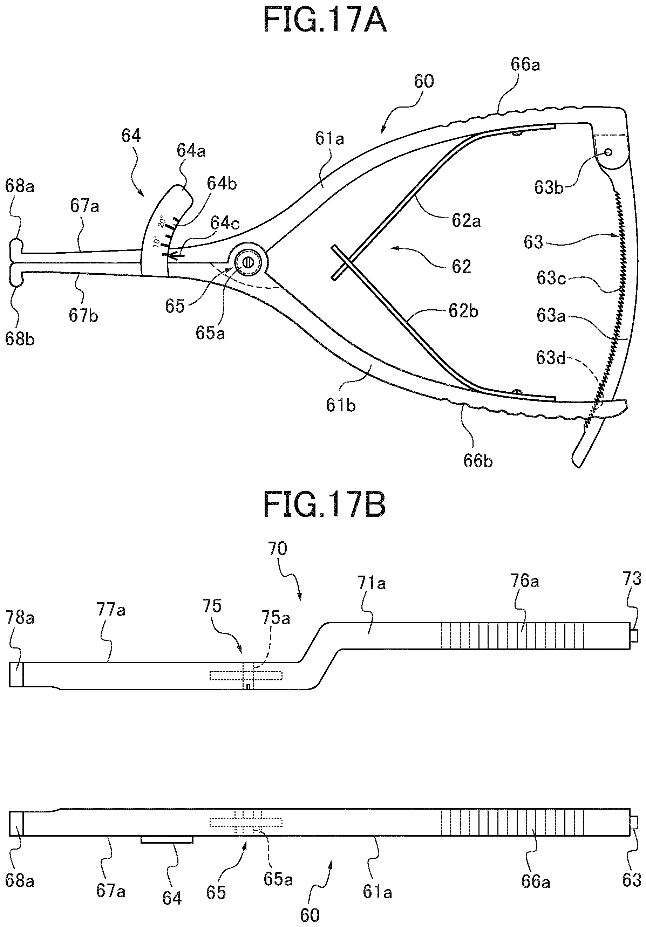

(Eighth Embodiment) Next, a spreader according to an eighth embodiment will be described with reference to FIGS. 17A and 17B. As shown in FIGS. 17A and 17B, the spreader in the eighth embodiment is a variation of the third embodiment shown in FIGS. 10A to 12 and the sixth embodiment with the first and second opening and closing mechanisms 60, 70 shown in FIGS. 15A and 15B. The eighth embodiment includes the first opening and closing mechanism 60 including the straight handles 66a, 66b without offsetting similar to the third embodiment, and the second opening and closing mechanism 70 including the offset handles 76a, 76b similar to the sixth embodiment. According to the above structures, the handles 76a, 76b of the second opening and closing mechanism 70 are arranged separated from the handles 66a, 66b of the first opening and closing mechanism 60 with a predetermined distance therebetween when the operation portions 67a, 67b, 77a, 77b are arranged in parallel adjacent to each other. This predetermined distance is a half of the distance between the handles 66a, 66b, 76a, 76b in the sixth embodiment.

The first opening and closing mechanism 60 and the second opening and closing mechanism 70 are not limited to the above structures. The first opening and closing mechanism 60 may include the offset handles 66a, 66b similar to the sixth embodiment while the second opening and closing mechanism 70 may include the straight handles 76a, 76b similar to the third embodiment.

According to the spreader of the eighth embodiment configured as above, the first and second blades 10, 20 can be collectively or individually opened and closed at a predetermined angle or angles by collectively or individually operating the first and second opening and closing mechanisms 60, 70. In addition, offsetting the handles 76a, 76b of the second opening and closing mechanism 70 makes it easier to secure the field of view and the space of the surgical field on the straight line from the affected part and reduces protrusions in the arrangement directions.

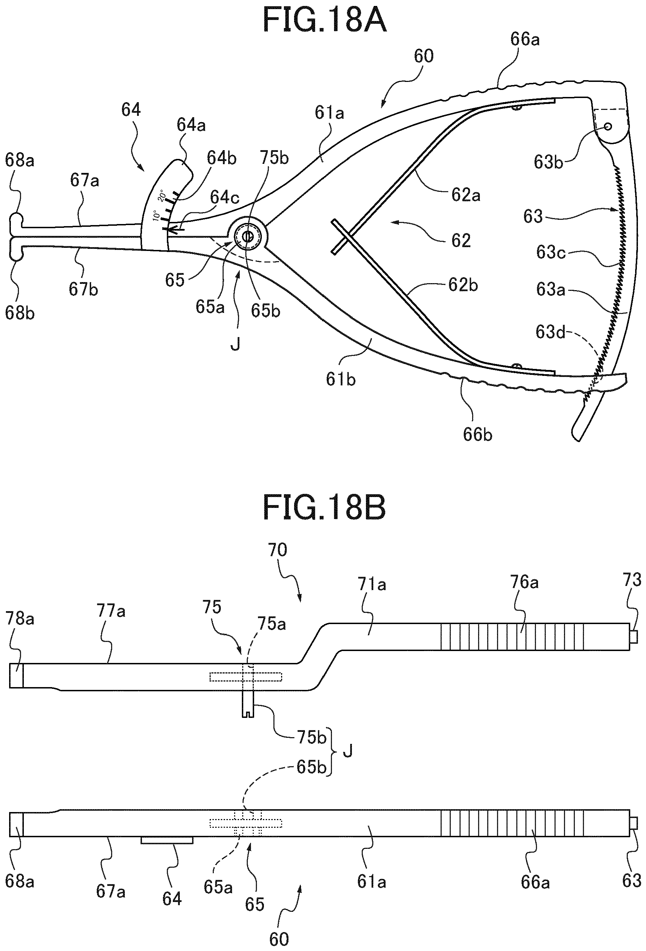

(Ninth Embodiment) Next, a spreader according to a ninth embodiment will be described with reference to FIGS. 18A and 18B. As shown in FIGS. 18A and 18B, the spreader of the ninth embodiment has a basic configuration similar to the spreader of the eighth embodiment including the first and second opening and closing mechanisms 60, 70 shown in FIGS. 17A and 17B except that the first and second opening and closing mechanisms 60, 70 are provided with the engagement mechanism J that connects mechanisms 60, 70 and engages them with each other. The engagement mechanism J in the ninth embodiment includes the engagement protrusion 75b and the engagement recess 65b. Similar to the fourth embodiment, the engagement protrusion 75b is provided on the connection shaft 75a of the second opening and closing mechanism 70 to extend therefrom. The engagement recess 65b is provided within the cylindrical connection shaft 65a of the first opening and closing mechanism 60. It should be noted that the first and second opening and closing mechanisms 60, 70 may be inseparably connected by an appropriate connection mechanism in the ninth embodiment.

According to the spreader of the ninth embodiment configured as above, the first and second blades 10, 20 can be collectively or individually opened and closed at a predetermined angle or angles by collectively or individually operating the first and second opening and closing mechanisms 60, 70. Further, offsetting the handles 76a, 76b of the second opening and closing mechanism 70 makes it easier to secure the field of view and the space of the surgical field on the straight line from the affected part and reduces protrusions in the arrangement directions. Moreover, the engagement mechanism J can easily and accurately position the first and second opening and closing mechanisms 60, 70 when arranged the mechanisms 60, 70 in parallel adjacent to each other. The engagement mechanism J can prevent the first and second opening and closing mechanisms 60, 70, and the like from unexpectedly falling and improve stability and transportability during the operations.

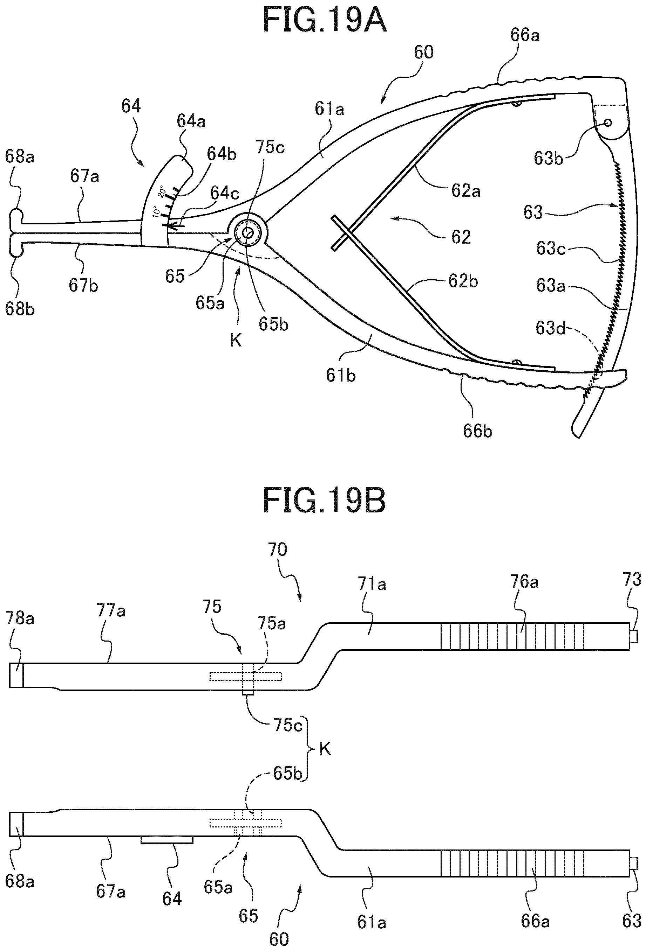

(Tenth Embodiment) Next, a spreader according to a tenth embodiment will be described with reference to FIGS. 19A and 19B. As shown in FIGS. 19A and 19B, the spreader of the tenth embodiment has a basic configuration similar to that of the spreader of the sixth embodiment including the first and second opening and closing mechanisms 60, 70 shown in FIGS.15A and 15B, except that the spreader includes a positioning portion K. Hereinafter, the configuration of the positioning portion K different from the sixth embodiment will be mainly described. The positioning portion K includes a positioning protrusion 75c and a positioning recess 65c. The positioning protrusion 75c is provided on the connection shaft 75a of the second opening and closing mechanism 70 to extend toward the first opening and closing mechanism 60. The positioning recess 65c is provided within the cylindrical connection shaft 65a of the first opening and closing mechanism 60. The positioning protrusion 75c is shorter and smaller than the engagement protrusion 75b in the embodiment such as the ninth embodiment. Accordingly, the positioning protrusion 75c easily engages with the positioning recess 65c when the first and second opening and closing mechanisms 60, 70 are arranged adjacent to each other. In addition, the positioning protrusion 75c easily disengages from the positioning recess 65c by slightly sliding the first and second opening and closing mechanisms 60, 70 in the longitudinal direction or the axial direction.

According to the spreader of the tenth embodiment configured as above, the first and second blades 10, 20 can be collectively or individually opened and closed at a predetermined angle or angles by collectively or individually operating the first and second opening and closing mechanisms 60, 70. Further, offsetting the handles 66a, 66b, 76a, 76b of the first and second opening and closing mechanisms 60, 70 makes it easier to secure the field of view and the space of the surgical field on the straight line from the affected part. Moreover, the positioning portion K can easily and accurately position the first and second opening and closing mechanisms 60, 70 when the mechanisms 60, 70 are arranged in parallel adjacent to each other. In addition, when one of the first and second opening and closing mechanisms 60, 70 is removed from the spreader body 30, the first and second opening and closing mechanisms 60, 70 can be easily separated from each other and compactly stored. In this embodiment, the handles 66a, 66b, 76a, 76b are the offset handles but may not limit thereto. The handles 66a, 66b, 76a, 76b may be the straight handles or the combination of the offset and straight handles.

Hereinafter, the effects of the embodiments of the present disclosure will be described. The spreader 100, 100A, 100B, etc. in the above embodiments includes the spreader body 30 including the first blade 10 and the second blade 20 that is arranged in parallel to the first blade 10, the opening and closing mechanism 40, 60, 70, 80 that is configured to open and close the first and second blades 10, 20. The first blade 10 and the second blade 20 respectively include the arms 11a, 11b, 21a, 21b that are connected at tips thereof to rotate in directions away from each other and directions close to each other, and the angle adjuster 12 that is configured to adjust the angle between the arms 11a, 11b, 21a, 21b.

The opening and closing mechanism 40, 60, 70, 80 includes the shafts 41a, 41b, 61a, 61b, 71a, 71b, 81a1, 81a2, 81b that are connected by the connection shaft 45a, 65a, 75a, 85a. Each of the shafts 41a, etc. includes the handle 46a, 46b, 66a, 66b, 76a, 76b, 86a1, 86a2, 86b at a first end and the operation portions 47a, 47b, 67a, 67b, 77a, 77b, 87a1, 87a2, 87b at a second end that are operated in conjunction with the handle 46a, etc. The connection shaft 45a, etc. is located between the first end and the second end. The operation portion 47a, etc. of the shaft 41a, etc. includes an engagement portion (engagement protrusions 48a, 48b, 68a, 68b, 78a, 78b, 88a1, 88a2, 88b) that engages with the engagement recess 234a, etc. or the base end portion 14a, 14b of the first or second arm 11a, 11b of the first blade 10 and/or the base end portion 24a, 24b of the first or second arm 21a, 21b of the second blade 20.

According to the above configuration, the engagement of the base end portion 14a, etc. with the engagement portion makes it possible to open the first and second blades 10, 20 only by the surgeon moving the handles 46a, etc. in the directions close to each other. Thereby, the cut section 2 can be easily and reliably enlarged to a desired size. Also, the blades 10, 20 can be smoothly and easily opened and closed in a collective or individual manner. Accordingly, the spreader 100, 100A, etc. having a simple configuration with excellent operability and durability can be provided.

The spreader 100, 100A of the first and second embodiments includes the spreader body 30 including the first blade 10 and the second blade 20 that is arranged in parallel to the first blade 10, and the opening and closing mechanism 40 that is configured to open and close the first blade 10 and the second blade 20. The first blade 10 and the second blade 20 respectively include the arms 11a, 11b, 21a, 21b that are connected at tips to rotate in directions away from each other and directions close to each other, and the angle adjuster 12 that is configured to adjust the angles between the arms 11a, 11b, 21a, 21b.

The opening and closing mechanism 40 includes a pair of shafts 41a, 41b that are rotatably connected by the connection shaft 45a. Each of the shafts 41a, 41b includes the handles 46a, 46b at the first end and the operation portions 47a, 47b, at the second end, that are configured to move in conjunction with the handles 46a, 46b. The connection shaft 45a is located between the first end and the second end. The operation portion 47a of the first shaft 41a includes a first engagement portion (engagement recess 48a) that engages with the base end portions 14a, 24a of the first arms 11a, 21a of the first and second blades 10, 20. The operation portion 47b of the second shaft 41b includes a second engagement portion (engagement recess 48b) that engages with the base end portions 14b, 24b of the second arms 11b, 21b of the first and second blades 10, 20.

According to the above configuration, the engagement of the base end portions 14a, 14b, 24a, 24b with the engagement portions makes it possible to open the first and second blades 10, 20 at one time only by the surgeon moving the handles 46a, 46b in the directions close to each other. Further, the angles θ1, θ2 between the arms 11a, 11b, 21a, 21b can be easily adjusted to the same angle at one time. Thereby, the cut section 2 can be easily and reliably enlarged to a desired size. Also, the blades 10, 20 can be smoothly and easily opened and closed in a collective or individual manner. Accordingly, the spreader 100, 100A, etc. having a simple configuration with excellent operability and durability can be provided.

The fifth embodiment includes one opening and closing mechanism 80. The opening and closing mechanism 80 includes, as a plurality of shafts, a support shaft 81b and two operation shafts 81a1, 81a2 that are arranged in parallel and rotatably connected to the support shaft 81b via the connection shaft 85a. The support shaft 81b and the two operation shafts 81a1, 81a2 respectively include the handle 86b, 86a1, 86a2 at the first end and the operation portion 87b, 87a1, 87a2, at the second end, that is configured to move in conjunction with the handle 86b, etc. and includes an engagement portion (engagement protrusion 88b, 88a1, 88a2), respectively. The connection shaft 85a is located between the first end and the second end. The engagement portion of the operation portion 87b of the support shaft 81b engages with the base end portions of the first arms of the first and second blades 10, 20. The engagement portion of the first one of the operation shafts 81a1, 81a2 engages with the base end portion of the second arm of the first blade 10. The engagement portion of the second one of the operation shafts 81a1, 81a2 engages with the base end portion of the second arm of the second blade 20. According to the above configuration, the first and second blades 10, 20 of the spreader body 30 can be collectively or individually opened and closed by collectively or individually operating the first operation shaft 81a1 or the second operation shaft 81a2.

In the third, fourth, sixth to tenth embodiments, two opening and closing mechanisms 60, 70 arranged in parallel are provided. Each of the opening and closing mechanisms 60, 70 includes a pair of shafts 61a, 61b, 71a, 71b that are rotatably connected by the connection shaft 65a, 75a. The engagement portions provided in the pair of shafts of the first one of the opening and closing mechanisms engage with the base end portions 14a, 14b of the arms 11a, 11b of the first blade 10. The engagement portions provided in the pair of shafts of the second one of the opening and closing mechanisms engage with the base end portions 24a, 24b of the arms 21a, 21b of the second blade 20. With this configuration, the first and second blades 10, 20 can be collectively and individually opened and closed by operating collectively and individually the first and second opening and closing mechanisms 60, 70. Moreover, the angles θ1, θ2 of the first and second blades 10, 20 can be easily and arbitrarily adjusted.

The opening and closing mechanisms 60, 70 may be configured to be separably engaged with each other by the engagement mechanism J at least at the locations of the connection shafts 65a, 75a. Alternatively, the opening and closing mechanisms 60, 70 may be configured to be inseparably engaged with each other by a connection mechanism at least at the locations of the connection shafts 65a, 75a. According to the above configurations, the opening and closing mechanisms 60, 70 can be easily and accurately positioned when arranged in parallel adjacent to each other. In addition, the opening and closing mechanisms 60, 70 can be attached to the first and second blades 10, 20 efficiently and stably.

Also, the tenth embodiment includes the positioning portion K for arranging the opening and closing mechanisms 60, 70 in parallel. With this configuration, the opening and closing mechanisms 60, 70 can be easily and accurately positioned when arranged in parallel adjacent to each other. Further, the first and second opening and closing mechanisms 60, 70 can be easily disengaged from each other and compactly stored.

In the sixth to tenth embodiments, at least one of the handles of the opening and closing mechanism offsets relative to the operation portion. This configuration makes it easier to secure the field of view and the space of the surgical field on the straight line from the affected part.

Also, in the above embodiments, the connection portion includes the engagement protrusions 48a, 48b, 68a, 68b, 78a, 78b, 88a1, 88a2, 88b each of which extends from the tip of the operation portion 47a, 47b, 67a, 67b, 77a, 77b, 87a1, 87a2, 87b in the opening direction of the arm 11a, 11b, 21a, 21b. Each of the base end portions 14a, 14b, 24a, 24b of the arms 11a, 11b, 21a, 21b of the first blade 10 and/or the second blade 20 includes the engagement recess 134a, 134b, 234a, 234b with which the engagement protrusion 48a, 48b, 68a, 68b, 78a, 78b, 88a1, 88a2, 88b engages. According to this configuration, the gripping force applied to the handles 46a, 46b, 66a, 66b, 76a, 76b, 86a1, 86a2, 86b can be efficiently transmitted to the spreader body 30 and the spreader body 30 can be easily opened without excessive power, labor, etc.

In addition, each of the angle adjusters 12, 22 in the above embodiments includes the screw hole 12a, 22a provided in the first arm 11a, 21a to extend through the first arm 11a, 21a, and the set screw 12b, 22b that is inserted into the screw hole 12a, 22a and abuts the inner surface of the second arm 11b, 21b. The angle adjusters 12, 22 can maintain the open state of the arms 11a, 11b, 21a, 21b at a desired angle or angles θ1, θ2 even when the opening and closing mechanism 40 is removed.

Also, the spreader body 30 in the above embodiments includes the angle scale member 18 that is provided in the first blade 10 (and/or second blade 20) to indicate the angle between the arms 11a, 11b. The angle scale member 18 includes the plate 18a that is attached to the inner surface of the first arm 11a at one end thereof and extends toward the second arm 11b, the angle scale marks 18b that are provided on the plate 18a, the insertion hole 18c that is provided in the second arm 11b and into which the plate 18a is removably inserted, the window 18d through which the angle scale marks 18b are visible, and the needle 18e that points to one of the angle scale marks 18b in accordance with the angle between the arms 11a, 11b. This configuration makes it possible for the surgeon to easily and clearly know the angle θ1 between the arms 11a, 11b and the enlarged state of the cut section 2. Further, the misalignment and/or displacement of the angle scale member 18 is suppressed so that the angle θ1 can always be measured accurately. Moreover, the first blade 10 and the second blade 20 can be stably arranged adjacent to each other since the angle scale member 18 does not protrude from the front and back surfaces of the first blade 10. As a result, excellent operability can be maintained.

Also, in the above embodiments, the scale (insertion depth scale 19) that indicates the insertion depth into the cut section 2 of the bone 1 is provided on the outer surface (front surface) parallel to the surface where the angle scale marks 18b of the angle scale member 18 of the first blade 10 (and/or second blade 20) are provided. Thereby, the insertion depth of the spreader body 30 into the cut section 2 can be easily and accurately grasped without using a separate gauge.

Further, in the above embodiments, the opening and closing mechanism 40, 60, 70, 80 includes the angle scale member 44, 64, 84 that indicates the angle between the operation portions 47a, 47b, 67a, 67b, 77a, 77b, 87a1, 87b. The angle scale member is disposed substantially on the same plane as the angle scale member 18 of the spreader body 30. According to the above configuration, the surgeon or practitioner can see the angle scale member 44, 64, 74, 84 and the angle scale member 18 from the same direction. Therefore, the posture during the operation can be maintained stably, and accordingly, the operation can be smoothly and efficiently performed.

In addition, the above embodiments include the drive member 50 for striking or driving the first blade 10 and the second blade 20 into the cut section 2 of the bone 1. The drive member 50 includes the grip portion 51 to be held by the surgeon, and the receiving portion 52 that collectively receives the base end portions 14a, 14b, 24a, 24b of the first and second blades 10, 20. According to the above configuration, the first and second blades 10, 20 can be stably held by the drive member 50, and the striking operation into the cut section 2 can be performed efficiently.

The various embodiments of the present disclosure have been described with reference to the drawings. The above embodiments only exemplify the present disclosure and accordingly the present disclosure is not limited to the above embodiments. Any design changes or modifications that do not deviate from the gist of the present disclosure are included in the present disclosure.

### CROSS-REFERENCE TO RELATED APPLICATION

This application is based upon and claims the benefit of priority from Japanese Patent Application No. 2018-244498 filed on December 27, 2018, and Japanese Patent Application No. 2019-156090 filed on August 28, 2019, the entire disclosure of which are incorporated herein by reference.

## Claims

1. A spreader comprising:
a spreader body that comprises a first blade and a second blade that is arranged in parallel with the first blade; and
an opening and closing mechanism that is configured to open and close the first blade and the second blade;
wherein each of the first blade and the second blade comprises:
a first arm and a second arm that are connected at tips to rotate in a direction away from each other and a direction close to each other, and
an angle adjuster that is configured to adjust an angle between the first arm and the second arm;
wherein the opening and closing mechanism comprises a plurality of shafts that are rotatably connected by a connection shaft,
wherein each of the plurality of shafts comprises a handle at a first end and an operation portion at a second end, the operation portion being configured to be operated in conjunction with the handle, and the connection shaft being located between the first end and the second end, and
wherein the operation portion of each of the plurality of shafts comprises an engagement portion that is configured to engage with a base end portion of the first arm or the second arm of the first blade and/or a base end portion of the first arm or the second arm of the second blade.

2. The spreader according to claim 1,
wherein the opening and closing mechanism is a single opening and closing mechanism,
wherein the opening and closing mechanism comprises first and second shafts that are rotatably connected by the connection shaft,
wherein the engagement portion of the operation portion of the first shaft is configured to engage with the base end portions of the first arms of the first and second blades, and the engagement portion of the operation portion of the second shaft is configured to engage with the base end portions of the second arms of the first and second blades.

3. The spreader according to claim 1,
wherein the opening and closing mechanism is a single opening and closing mechanism,
wherein the opening and closing mechanism comprises, as the plurality of shafts,
a support shaft, and
first and second operation shafts that are arranged in parallel and rotatably connected to the support shaft by the connection shaft,
wherein each of the support shaft and the first and second operation shafts comprises a handle at a first end, and an operation portion at a second end, the operation portion being configured to be operated in conjunction with the handle and comprising an engagement portion, the connection shaft being located between the first end and the second end,
wherein the engagement portion of the operation portion of the support shaft is configured to engage with the base end portions of the first arms of the first and second blades,
wherein the engagement portion of the operation portion of the first operation shaft is configured to engage with the base end portion of the second arm of the first blade, and the engagement portion of the operation portion of the second operation shaft is configured to engage with the base end portion of the second arm of the second blade.

4. The spreader according to claim 1, further comprising a second opening and closing mechanism that is arranged in parallel with the opening and closing mechanism,
wherein each of the opening and closing mechanisms comprises first and second shafts that are rotatably connected by the connection shaft,
wherein the engagement portions of the operation portions of the first and second shafts of the opening and closing mechanisms are configured to engage with the base end portions of the first and second arms of the first blade, and the engagement portions of the operation portions of the first and second shafts of the second opening and closing mechanism are configured to engage with the base end portions of the first and second arms of the second blade.

5. The spreader according to claim 4, further comprising an engagement mechanism that is configured to separably engage the two opening and closing mechanisms at least at a location of the connection shaft.

6. The spreader according to claim 4, further comprising a connection mechanism that is configured to inseparably connect the two opening and closing mechanisms at least at a location of the connection shaft.

7. The spreader according to one of claims 4 to 6, further comprising a positioning portion that is configured to arrange the two opening and closing mechanisms in parallel.

8. The spreader according to one of claims 4 to 7, wherein the handle of at least one of the opening and closing mechanisms offsets with respect to the operation portion.

9. The spreader according to one of claims 1 to 8,
wherein the engagement portion comprises an engagement protrusion that extends from a tip in an opening direction of each of the first and second arms, and
wherein each of the base end portions of the first and second arms of the first blade and/or the second blade comprises an engagement recess with which the engagement protrusion engages.

10. The spreader according to one of claims 1 to 9, wherein the angle adjuster comprises:
a screw hole that is provided in the first arm to extend through the first arm; and
a set screw that is inserted into the screw hole and abuts an inner surface of the second arm.

11. The spreader according to one of claims 1 to 10,
wherein the spreader body comprises an angle scale member that is provided in at least one of the first blade and the second blade to indicate an angle between the first and second arms, and
wherein the angle scale member comprises:
a plate that is attached to an inner surface of the first arm at one end thereof and extends toward the second arm;
angle scale marks that are provided on the plate;
an insertion hole that is provided in the second arm and into which the plate is removably inserted;
a window through which the angle scale marks are visible; and
a needle that points to one of the angle scale marks in accordance with an angle between the first and second arms.

12. The spreader according to claim 11, further comprising scale marks that indicate an insertion depth into a cut section of a bone, and
wherein the scale marks are provided on an outer surface of at least one of the first and second blades, the outer surface being parallel to a surface on which the angle scale marks of the angle scale member are provided.

13. The spreader according to claim 11 or 12, wherein the opening and closing mechanism comprises an angle scale member that is disposed substantially on a same plane as the angle scale member of the spreader body to indicate an angle between the operation portions.

14. The spreader according to one of claims 1 to 13, further comprising a drive member for striking the first and second blades into a cut section of a bone,
wherein the drive member comprises:
a grip portion that is held by a surgeon; and
a receiving portion that collectively receives the base end portions of the first and second blades.
